# EUROPEAN PATENT APPLICATION

(11) **EP 2 213 290 A1**
(43) Date of publication of application: **04.08.2010**
(21) Application number: 08855391.2
(22) Date of filing: 28.11.2008
(51) Int. Cl.: A61K 31/553, A61K 31/198, A61K 45/00, A61P 25/14, A61P 25/16, A61P 43/00, C07D 413/14

(54) **AGENT FOR IMPROVING MOTOR COMPLICATIONS OR PSYCHIATRIC SYMPTOMS IN PARKINSON'S DISEASE**

(30) Priority: 30.11.2007 JP 2007311189
(71) Applicant: Asubio Pharma Co., Ltd., Minato-ku Tokyo 107-8541 (JP)
(72) Inventor: TANI, Yoshihiro, Ibaraki-shi Osaka 567-0893 (JP); KOYAMA, Makoto, Takatsuki-shi Osaka 569-0814 (JP)
(74) Representative: Stoner, Gerard Patrick
(86) International application number: PCT/JP2008/072105
(87) International publication number: WO 2009/069828

(57) **Abstract**

A drug containing a compound having the formula (I): 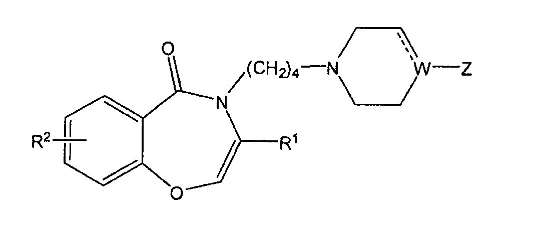 or a pharmacologically acceptable salt thereof or their hydrates, which alleviates motor complications associated with a treatment with levodopa for Parkinson's disease, delays the onset of motor complications associated with a treatment with levodopa, and inhibiting or delaying the advance of symptoms of Parkinson's disease is provided. The compound having the formula (I) has a serotonin 1A receptor partial agonist action, does not have an antagonist action against dopamine D2 receptors, has an agonist action against dopamine D3 receptors, has an effect of alleviation and delay of onset of motor complications associated with repeated doses of levodopa and, further, is also effective against associated with psychiatric symptoms in advanced stage Parkinson's disease patients.

## Description

### TECHNICAL FIELD

The present invention relates to a drug for alleviating the motor complications associated with a treatment with levodopa for Parkinson's disease, delaying the onset of motor complications associated with a treatment with levodopa, and inhibiting or delaying the advance of symptoms of Parkinson's disease. The present invention further relates to a drug for alleviating the psychiatric symptoms accompanying advanced stage Parkinson's disease.

### BACKGROUND ART

Parkinson's disease is a neurodegenerative disorder presenting resting tremor, rigidity, akinesia, and disorder of postual reflex as main symptoms. Parkinson's disease is classified into early stage Parkinson's disease and advanced stage Parkinson's disease. That is, early stage Parkinson's disease indicates the disease state at the relatively early onset where levodopa and dopamine receptor agonists are not yet used, while advanced stage Parkinson's disease indicates the disease state where levodopa is already being taken and further the various problems accompanied with the long-term use are appearing. The Japanese Society of Neurology Treatment Guidelines ("Japanese Society of Neurology Treatment Guidelines: Parkinson's Disease Treatment Guideline 2002", Ad Hoc Committee, Rinsho Shinkeigaku (Clinical Neurology), 2002, 42, p. 430-94) describe guidelines for treatment of Parkinson's disease based on efficacy of treatment drugs and treatment methods and on safety divided into the early stage and advanced stage.

For Parkinson's disease, levodopa (chemical name: L-3,4-dihydrophenylalanine, also called "L-dopa") is the most effective treatment drug. This is because Parkinson's disease is a progressive neurological disease occurring due to the specific degeneration and loss of nigrastriatal dopamine neuron of the midbrain and the main treatment for it is believed to be replenishment of the insufficient dopamine. However, levodopa causes problems, called motor complications, in Parkinson's disease patients along with long-term administration and, therefore, makes treatment difficult. This is the state called "advanced stage Parkinson's disease". "Motor complications" is a frequently used expression including dyskinesia interpreted as being based on the excessive action of levodopa and the daily fluctuations and motor fluctuations of Parkinson's disease symptoms, that is, the wearing off phenomenon, on-off phenomenon, and no-on/delayed on phenomenon ("Japanese Society of Neurology Treatment Guidelines: Parkinson's Disease Treatment Guideline 2002", Ad Hoc Committee, Rinsho Shinkeigaku (Clinical Neurology), 2002, 42, p. 430-94). Dyskinesia is involuntary motion related to levodopa (seen in mouth, tongue, face, limbs, and body trunk). It includes peak-dose dyskinesia appearing when the concentration of levodopa in the blood is high and diphasic dyskinesia appearing biphasically in the period of increase and the period of decrease of the concentration of levodopa in the blood. Both are symptoms showing excessive levodopa. Further, from the sense of being levodopa induced, the expression "LID (levodopa-induced dyskinesia)" is also frequently used. The "wearing off phenomenon" means the phenomenon where the period of pharmacological effect of the levodopa is shortened and the effect of the levodopa wears off after the elapse of several hours after administration of levodopa. The "on-off phenomenon" is the phenomenon where, regardless of the time of administration of the levodopa, the symptoms are alleviated (on) and suddenly become worse (off). The "noon phenomenon" is the phenomenon where no effect is exhibited even if levodopa is administered, while the "delayed on phenomenon" is the phenomenon where time is required for the effect of levodopa to be expressed. These phenomena are all fluctuations of the levodopa on time (i.e., period during which effects of levodopa are seen) and conversely the off time (i.e., period where effects of levodopa are insufficient and Parkinson's disease symptoms appear), and therefore, are called "daily fluctuations" or "motor fluctuations" of Parkinson's disease symptoms ("Japanese Society of Neurology Treatment Guidelines: Parkinson's Disease Treatment Guideline 2002", Ad Hoc Committee, Rinsho Shinkeigaku (Clinical Neurology), 2002, 42, p. 430-94). When the motor complications of Parkinson's disease appear, recovery cannot be expected even if switching to another drug. For this reason, it has been reported to start treatment at the early stage of onset of Parkinson's disease not with levodopa, but with a dopamine D2 receptor agonist so as to reduce the risk of advanced stage motor complications, but it is difficult to obtain a sufficient therapeutic effect only with a dopamine D2 receptor agonist. Sooner or later, combined use with levodopa becomes necessary and motor complications are experienced (Murata Miho, "The therapy of wearing-off", Nippon Rinsho (Japanese Journal of Clinical Medicine), 2004, 62, p. 1716-1719). Therefore, the motor complications associated with a treatment with levodopa remain a major problem in the treatment of Parkinson's disease.

The main metabolizing enzyme of levodopa in the body is dopa decarboxylase, but the metabolism of levodopa and dopamine also involves monoamine oxidase (MAO) and catechol-o-methyl transferase (COMT). In Parkinson's disease, inhibitors of these metabolizing enzymes increase the rate of utilization of levodopa and thereby promise to have an effect on the shortening of the period of pharmacological effect of levodopa in motor complications, that is, the wearing off phenomenon. Monoamine oxidase (MAO) comes in subtypes of Type A and Type B, but in the human striatum, the Type B is prevalent, so monoamine oxidase type B inhibitor (MAO-B) is useful. In clinical studies, the effectiveness against the wearing off phenomenon of selegiline is reported (Golbe L.I. et al., "Deprenyl in the treatment of symptom fluctuations in advanced Parkinson's disease", Clinical Neuropharmacology, 1988, 11, p. 45-55). Selegiline specifically inhibits monoamine oxidase B (MAO-B) and thereby obstructs the metabolism of dopamine in the striatum and enhances the effect of levodopa. On the other hand, the catechol-o-methyl transferase (COMT) inhibitor entacapone obstructs the pathway by which levodopa is metabolized to 3-o-methyl dopa (3-OMD) by catechol-o-methyl transferase in the peripheral organs and thereby delays the loss of levodopa from the peripheral organs and maintains the migration of levodopa to the brain and exhibits effectiveness against the wearing off phenomenon (Parkinson Study Group, "Entacapone improves motor fluctuations in levodopa-treated Parkinson's disease patients", Annals of Neurology, 1997, 42, p. 747-755). These enzyme inhibitors both exhibit efficacy in clinical studies by reinforcing and maintaining the effects of orally administered levodopa, but simultaneously may cause aggravation of the dyskinesia thought to be due to the excessive action of levodopa. In actuality, in clinical tests of entacapone, the most frequently observed side effect is reported to be the dyskinesia of motor complications (Mizuno Y. et al., "Placebo-controlled, double-blind dose-finding study of entacapone in fluctuating parkinsonian patients", Movement Disorders, 2007, 22, p. 75-80). Therefore, these metabolizing enzyme inhibitors cannot be said to meet the unmet needs of advanced stage Parkinson's disease patients.

Serotonin 1A receptor agonists are known to have an antidepressant, anti-anxiety effect, neuroprotective effect, etc., but recently the possibility of action in alleviating the above-mentioned motor complications associated with a treatment with levodopa for Parkinson's disease has been reported (Nicholson S.L. & Brotchie J.M., "5-Hydroxytryptamine (5-HT, serotonin) and Parkinson's disease - opportunities for novel therapeutics to reduce the problems of levodopa therapy", European Journal Neurology, 2002, 9, p. 1-6). Parkinson's disease is a disorder of the brain dopamine nervous system, yet why is a serotonin 1A receptor agonist rather than a dopamine receptor related drug effective for treatment of motor complications associated with a treatment with levodopa for Parkinson's disease. The scientific reasons are that (1) in Parkinson's disease patients exhibiting motor complications, a rise in the peak concentration of levodopa in the blood and the shortening of the half life have been reported (Murata M. et al., "Chronic levodopa therapy enhances dopa absorption: contribution to wearing-off", Journal of Neural Transmission, 1996, 103, p. 1177-1185), (2) the concentration of levodopa in the blood is closely correlated with the brain dopamine concentration, and therefore, it is predicted that, along with the advance of Parkinson's disease, the dopamine concentration in the brain will show sudden fluctuations (Murata M. et al., "Chronic levodopa therapy enhances dopa absorption: contribution to wearing-off", Journal of Neural Transmission, 1996, 103, p. 1177-1185), (3) levodopa administered from the periphery is not only taken into the dopamine nervous system, but also the serotonin nervous system, therefore the possibility of release converted to dopamine has been reported (Arai R. et al., "Immunohistochemical evidence that central serotonin neurons produce dopamine from exogenous L-DOPA in the rat, with reference to the involvement of aromatic L-amino acid decarboxylase", Brain Research, 1994, 667, p. 295-299, Yamada H. et al., "Immunohistochemical detection of L-DOPA-derived dopamine within serotonergic fibers in the striatum and the substantia nigra pars reticulate in parkinsonian model rats", Neuroscience Research, 2007, 59, p. 1-7), (4) there is a high possibility that the release of dopamine derived from levodopa taken into the serotonin nervous system is controlled (or inhibited) by serotonin 1A receptor agonists in the same way as serotonin (Carta M. et al., "Dopamine released from 5-HT terminals is the cause of L-DOPA-induced dyskinesia in parkinsonian rats", Brain, 2007, 130, p. 1819-1833). In actuality, clinical test results have been reported in which the serotonin 1A receptor agonist sarizotan (Olanow C.W. et al., "Multicenter, open-label, trial of sarizotan in Parkinson disease patients with levodopa-induced dyskinesias (the SPLENDID study)", Clinical Neuropharmacology, 2004, 27, p. 58-62), tandospirone (Kannari Kazuya et al., "Effect of selective 5-HT1A receptor agonist, tandospirone citrate in alleviating L-DOPA-induced dyskinesia", No To Shinkei (Brain & Nerve), 2002, 54, p. 133-137), and buspirone (Bonifati V. et al., "Buspirone in levodopa-induced dyskinesias", Clinical Neuropharmacology, 1994, 17, p. 73-82) exhibit effectiveness against motor complications accompanying levodopa therapy for Parkinson's disease. However, these drugs have not only a serotonin 1A receptor agonist action, but also an antagonist action with respect to dopamine D2 receptors. This action may be liable to lead to a weaker effect of levodopa in the treatment of Parkinson's disease. In actuality, in clinical tests, these drugs are reported to have the side effect of aggravation of Parkinson's disease symptoms (Olanow C.W. et al., "Multicenter, open-label, trial of sarizotan in Parkinson disease patients with levodopa-induced dyskinesias (the SPLENDID study)", Clinical Neuropharmacology, 2004, 27, p. 58-62, Kannari Kazuya et al., "Effect of selective 5-HT1A receptor agonist, tandospirone citrate in alleviating L-DOPA-induced dyskinesia", No To Shinkei(Brain & Nerve), 2002, 54, p. 133-137, Kleedorfer B. et al., "Buspirone in the treatment of levodopa induced dyskinesias", Journal of Neurology, Neurosurgery, and Psychiatry, 1991, 54, p. 376-377).

As a compound having an agonist action against both serotonin 1A receptors and dopamine D2 receptors, 7-(1-{[5-(4-fluorophenyl)pyridin-3-yl]methyl}piperidin-3-yl)-1,3-benzoxazol-2(3H)-one dihydrochloride is disclosed in Japanese Patent Publication No. 2005-298402A, but this compound is described as having increased the dopamine stimulus score, without increasing the dyskinesia score much at all, in evaluation of L-DOPA induced dyskinesia behavior in Parkinson's disease model rats (Japanese Patent Publication No. 2005-298402A), and is predicted as not satisfactorily alleviating both motor complications and Parkinson's disease symptoms.

Furthermore, serotonin 1A receptor agonists are classified from their form of actions into full agonists and partial agonists (partially activated weaker in action than full agonists), but the typical serotonin 1A receptor full agonist 8-OH-DPAT (chemical name: (R)-(+)-8-hydroxy-2-(di-n-propylamino)tetraline, hereinafter referred to as "8-OH-DPAT") exhibits an inhibitory action against dyskinesia induced by levodopa in Parkinson's disease model marmosets, but in high dosages reportedly is observed to weaken the therapeutic effect of levodopa (Iravani M.M. et al., "In 1-methyl-4-phenyl-1,2,3,6-tetrahydropyridine-treated primates, the selective 5-hydroxytryptamine la agonist (R)-(+)-8-OHDPAT inhibits levodopa-induced dyskinesia but only with/increased motor disability", The Journal of Pharmacology and Experimental Therapeutics, 2006, 319, p. 1225-1234). This means that it is difficult to set the suitable dosage for the drug used for levodopa therapy where the schedule of administration is set depending upon the symptoms of the patient. Further, with a full agonist, there is said to be an action causing amnesia. When considering long term administration, this is a problem to be concerned about.

In this way, in combined levodopa therapy of advanced stage Parkinson's disease patients, it is important not to detrimentally affect the treatment of Parkinson's disease with levodopa and to alleviate the problematic motor complications, but it has not been elucidated how to control the action on the dopamine nervous system and serotonin nervous system so as to obtain the desirable effects.

Further, Parkinson's disease is a progressive degenerative disorder of the nigrastriatal dopamine nerves, and therefore, it is believed that the condition worsens along with the advance of neurodegeneration. Therefore, if it were possible to delay or restore changes in the nigrastriatal dopamine nerves, a great therapeutic effect could be expected. In the Parkinson's disease model using animals, it is reported that a serotonin 1A receptor agonist has a nerve cell protective action and delays the onset of Parkinson's disease-like symptoms (Bezard E. et al., "5-HT1A receptor agonist-mediated protection from MPTP toxicity in mouse and macaque models of Parkinson's disease", Neurobiology of Disease, 2006, 23, p. 77-86). Further, dopamine D3 receptor agonists are reported to have a nerve cell protective action (Joyce J.N. & Millan M.J., "Dopamine D3 receptor agonists for protection and repair in Parkinson's disease", Current Opinion in Pharmacology, 2007, 7, p. 100-105). Recently, furthermore, the possibility of having a protective and reparative action on nigrastriatal dopamine nerves has also been suggested (Van Kampen J.M. & Eckman C.B., "Dopamine D3 receptor agonist delivery to a model of Parkinson's disease restores the nigrostriatal pathway and improves locomotor behavior", The Journal of Neuroscience, 2006, 26, p. 7272-7280). However, there is still no drug successfully treating Parkinson's disease by delaying or repairing neurodegeneration.

On the other hand, as one problem in the quality of life (QOL) of advanced stage Parkinson's disease patients, psychiatric symptoms may be mentioned. As non-motor symptoms of Parkinson's disease, it is reported that 60% or more of patients have psychiatric symptoms (Aarsland D. et al., "Range of neuropsychiatric disturbances in patients with Parkinson's disease", Journal of Neurology, Neurosurgery, and Psychiatry, 1999, 67, p. 492-496). Further, it is reported that about 40% of Parkinson's disease patients are in a depressed state. Depression is pointed to as a major factor inhibiting the quality of life of Parkinson's disease patients (Yamamoto Mitsutoshi, "Depression", Nippon Rinsho (Japanese Journal of Clinical Medicine), 2004, 62, p. 1661-1666). Furthermore, regarding the symptoms of depression in Parkinson's disease, it is reported that suicidal ideation, suicidal attempts, and other symptoms are rare, and therefore, this can be considered as a light depressed state or mood change different from the psychological disorder of major depression (Veazey C. et al., "Prevalence and treatment of depression in Parkinson's disease", The Journal of Neuropsychiatry and Clinical Neurosciences, 2005, 17, p. 310-323). Further, as a psychiatric symptom deteriorating along with the advance of Parkinson's disease, hallucinations are pointed to. Hallucinations are reportedly observed in about 20% of Parkinson's disease patients (Aarsland D. et al., "Range of neuropsychiatric disturbances in patients with Parkinson's disease", Journal of Neurology, Neurosurgery, and Psychiatry, 1999, 67, p. 492-496). According to the Japanese Society of Neurology Treatment Guidelines, when hallucinations appear, in principle the anti-Parkinson's disease drugs which had been used for treatment up to then are successively stopped, the prescription is simplified, and finally only levodopa is used for treatment. When the decrease in the amount of levodopa is difficult, it is prescribed that a small amount of an atypical antipsychotic be used ("Japanese Society of Neurology Treatment Guidelines: Parkinson's Disease Treatment Guideline 2002", Ad Hoc Committee, Rinsho Shinkeigaku (Clinical Neurology), 2002, 42, p. 430-94), but the side effects of atypical antipsychotics have also been pointed out and the control of Parkinson's disease symptoms becomes difficult, and therefore, there is a large effect on the quality of life of patients. The pathogenesis of hallucinations in Parkinson's disease patients is not clear, but it is hypothesized that this is because the dopamine receptors of the cortex are continually stimulated unphysiologically by excessive dopamine. Further, the possibility of abnormalities in the serotonin function being involved in the neurological symptoms has been pointed out (Melamed E. et al., "Involvement of serotonin in clinical features of Parkinson's disease and complications of L-DOPA therapy", Advances in Neurology, 1996, 69, p. 545-550). In actuality, it is reported that drugs inhibiting brain serotonin release alleviate neurological symptoms of advanced stage Parkinson's disease (Zoldan J. et al., "Psychosis in advanced Parkinson's disease: Treatment with ondansetron, a 5-HT3 receptor antagonist", Neurology, 1995, 45, p. 1305-1308). Furthermore, as behavioral disorders of Parkinson's disease patients, in addition to depression, anxiety and panic attacks have also been reported. These are sometimes seen synchronously with the off time and are taken note of in considering the disease conditions and treatment (Kashiwabara Kenichi, "Behavioral impairments", Nippon Rinsho (Japanese Journal of Clinical Medicine), 2004, 62, p. 1675-1678).

Accordingly, a drug for the treatment of Parkinson's disease for alleviating the motor complications becoming a problem in advanced stage Parkinson's disease patients and also effective against the accompanying psychiatric symptoms is desired.

On the other hand, International Patent Publication No. WO 96/24594 discloses that certain types of benzoxazepin derivatives have a strong affinity with the serotonin receptor 5-HT1A, exhibit a weak affinity with dopamine D2 receptors, exhibit an antianxiety action based on the indicator of anticonflict action, have suppressive activity in cerebral infraction and other brain protective actions in ischemic brain disorders in the transitory right middle cerebral artery occlusion (MCAO) model, and are useful for the treatment of anxiety neurosis, phobia, obsessive-compulsive disorder, schizophrenia, post-traumatic stress disorder, depressive neurosis, psychosomatic disorders, and other psychiatric and neurological disorders, eating disorders, menopausal disorders, infantile autism, and other disorders and other disorders involving the cerebral circulatory system accompanied with vomiting or cerebral occlusion and cerebral hemorrhaging. Further, Kamei K. et al., "New 5-HT1A Receptor Agonists Possessing 1,4-Benzoxazepine Scaffold Exhibit Highly Potent Anti-Ischemic Effects", Bioorganic & Medicinal Chemistry Letters, 2001, 11, p. 595-598, Kamei K. et al., "New piperidinyl- and 1,2,3,6-tetrahydropyridinyl-pyrimidine derivatives as selective 5-HT1A receptor agonists with highly potent anti-ischemic effects", Bioorganic & Medicinal Chemistry Letters, 2005, 15, p. 2990-2993, and Kamei K. et al., "Synthesis, SAR studies, and evaluation of 1,4-benzoxazepine derivatives as selective 5-HT1A receptor agonists with neuroprotective effect: Discovery of Piclozotan", Bioorganic & Medicinal Chemistry, 2006, 14, p. 1978-1992 disclose the structure-activity relationship of benzoxazepin derivatives exhibiting strong affinity with the serotonin receptor 5-HT1A and exhibiting weak affinity with dopamine D2 receptors.

### DISCLOSURE OF INVENTION

Under the above background, the object of the present invention is to provide a new drug for treatment of Parkinson's disease which is effective for the treatment and delay of onset of motor complications associated with a treatment with levodopa - a major problem in advanced stage Parkinson's disease -, effective against the psychiatric symptoms accompanying advanced stage Parkinson's disease as well, and promising the effect of delaying the advance of symptoms of Parkinson's disease.

In accordance with the present invention, there is provided a drug for alleviating the motor complications associated with a treatment with levodopa for Parkinson's disease comprising a compound expressed having the formula (I): 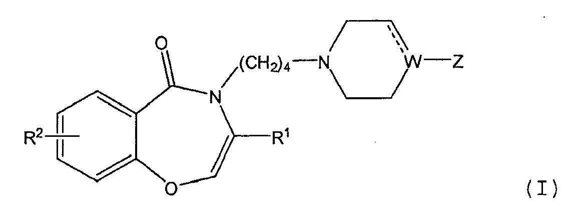 wherein R¹ indicates a hydrogen atom, chlorine atom or C₁ to C₄ alkyl group, R² indicates a hydrogen atom, halogen atom, methyl group or methoxy group, W indicates a nitrogen atom, CH or carbon atom, the dotted line indicates the absence of a bond when W is a nitrogen atom or CH and indicates the presence of a bond when W is a carbon atom, Z indicates a phenyl group, pyridyl group, or pyrimidinyl group which is unsubstituted or substituted with 1 to 3 substituent groups selected from the group consisting of a methyl group, methoxy group and halogen atom
or a pharmacologically acceptable salt thereof or their hydrates.

### BRIEF DESCRIPTION OF THE DRAWINGS

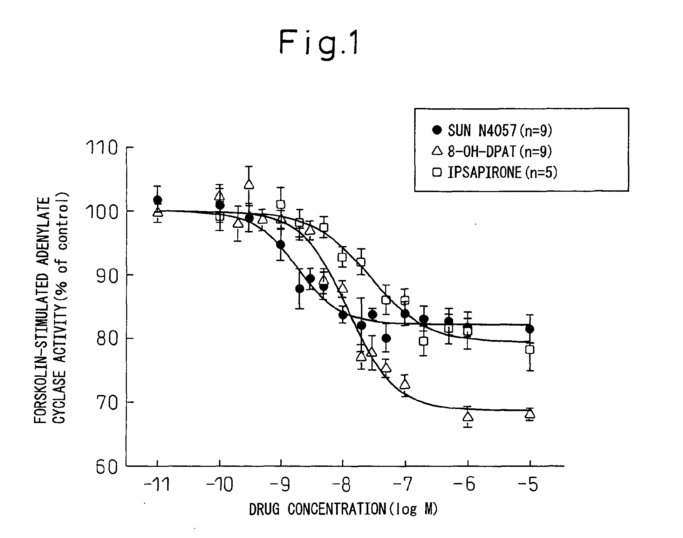

FIG. 1 is a view showing the effect of serotonin 1A receptor agonists against the adenylate cyclase activity in rat hippocampal membranes. The adenylate cyclase activity is measured using, as an indicator, the amount of cAMP produced by the same enzyme. The inhibition rate with the serotonin 1A receptor agonist is found using the amount of production due to forskolin (10 µM) stimulus as 100% and is shown by the mean value ± standard error (each group n=5-9).

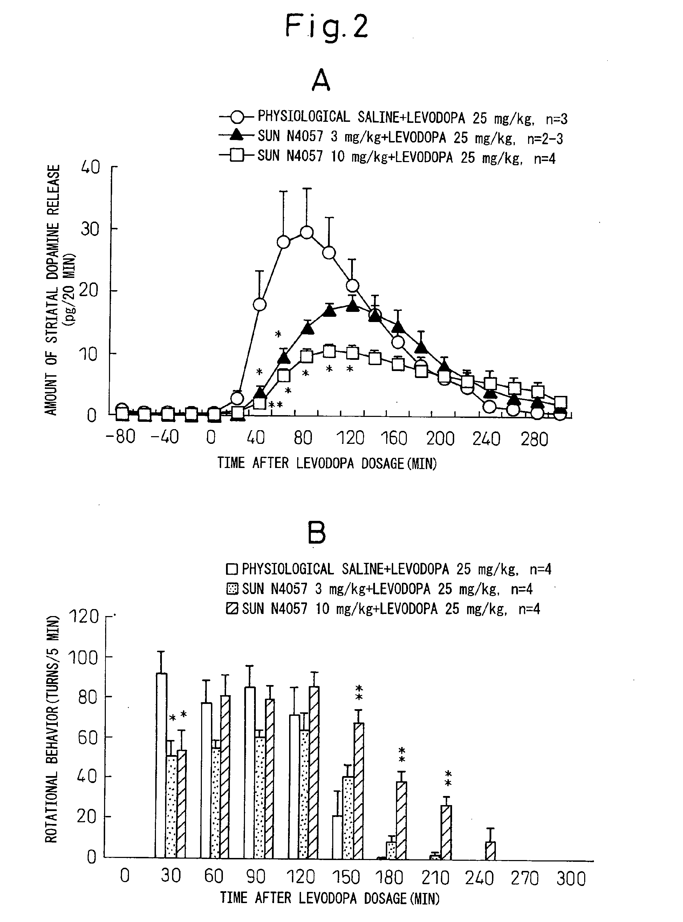

FIG. 2 is a view showing the effect of single intraperitoneal administration of SUN N4057 against levodopa-induced striatal dopamine release in Parkinson's disease model rats (i.e., week 6 of repeated doses of levodopa). The amount of release of dopamine from the rat striatum was measured using brain microdialysis, without anesthesia or physical restraint and the number of rotations was determined. That is, the amount of striatal dopamine release (A) was determined every 20 minutes from levodopa intraperitoneal administration until 5 hours elapsed, while the number of rotations in 5 minutes (B) was determined every 30 minutes from levodopa dosage. SUN N4057 (3, 10 mg/kg) was intraperitoneally administered 20 minutes before administration of the levodopa (25 mg/kg). The results are shown by the mean value ± standard error (each group, n=2-4). * p<0.05 and ** p<0.01: significant difference compared with physiological saline group (Dunnett's test).

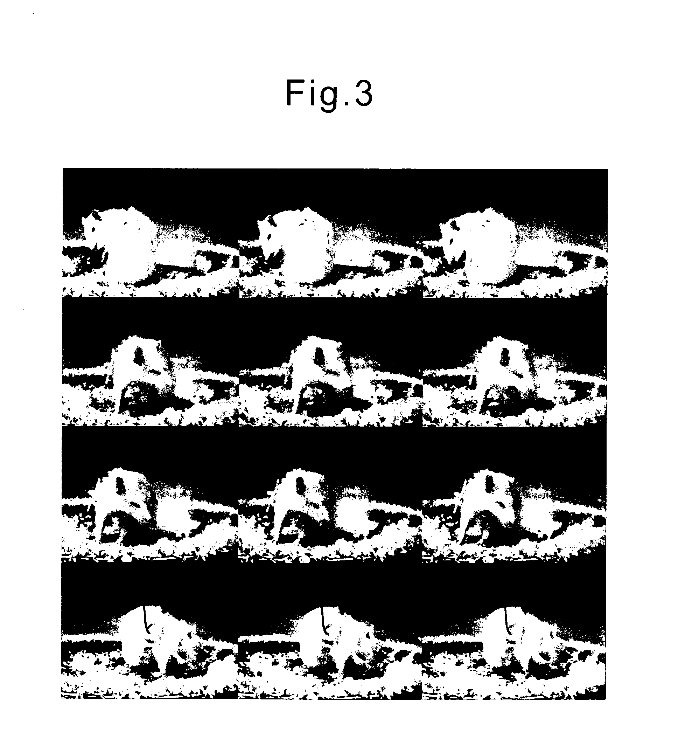

FIG. 3 is a view showing hyperkinesia of the forelimbs appearing due to 5 weeks of repeated doses of levodopa in Parkinson's disease model rats. Involuntary extending and other hyperkinesia occurred at the forelimb of the opposite side (i.e., left side) but not the destroyed side (i.e., right side). Note that the typical rat behavior was captured on video, then shown as consecutive shots.

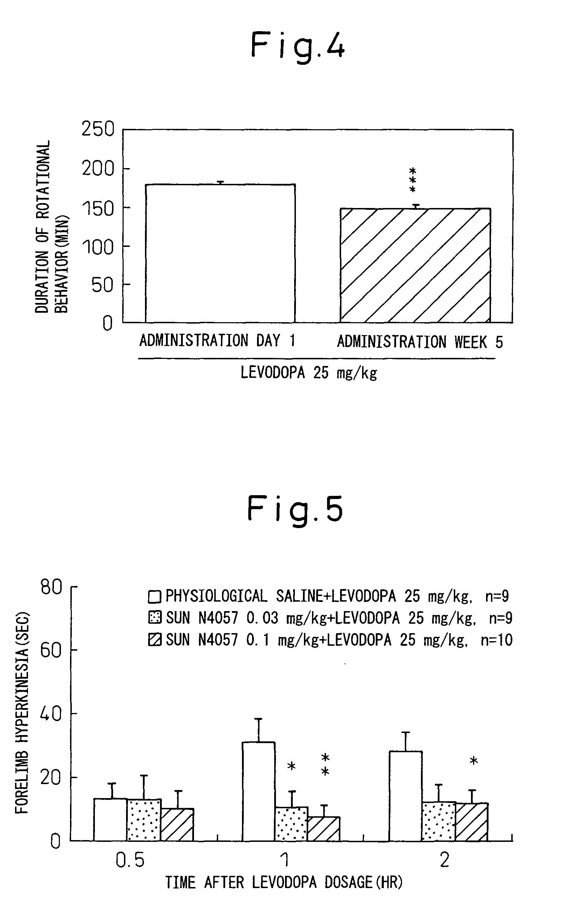

FIG. 4 is a view showing the change in the duration of rotational behavior accompanying repeated doses of levodopa of Parkinson's disease model rats. A comparison of day 1 of levodopa dose and week 5 of repeated doses is shown. On day 1 of levodopa dose and week 5 of repeated doses, a rotational behavior determining system was used to determine the number of rotations for every 5 minutes. The period during which each rat exhibited 20% or more of the maximum number of rotations was found and defined as the rotational behavior duration. The results are shown by the mean value ± standard error for 28 examples in each group. ***p<0.001: significant difference comparing day 1 of levodopa dose and week 5 of repeated doses (Wilcoxon rank test).
   Note that FIG. 4 showed the data before administration of SUN N4057 shown in FIGS. 5 and 6. Before administration of sarizotan shown in FIGS. 7 and 8 (37 examples in each group), before administration of the Example Compound 4 of Japanese Patent Publication (A) No. 2005-298402 shown in FIGS. 9 and 10 (27 examples in each group), and before administration of the SUN N4057 analogs (compound Ib and compound Ic) shown in FIGS. 13 and 14 (37 examples in each group), substantially the same results are obtained.
FIG. 5 shows the effects of SUN N4057 repeated subcutaneous administration on hyperkinesia of the forelimbs of Parkinson's disease model rats (week 7 of repeated doses of levodopa). The period of appearance of hyperkinesia of the forelimbs after levodopa dosage at week 7 of repeated doses of levodopa (week 2 of SUN N4057 repeated subcutaneous administration) was measured and the result was shown by the mean value ± standard error (each group n=9-10). * p<0.05, ** p<0.01: significant difference compared with physiological saline group (Dunnett's test).

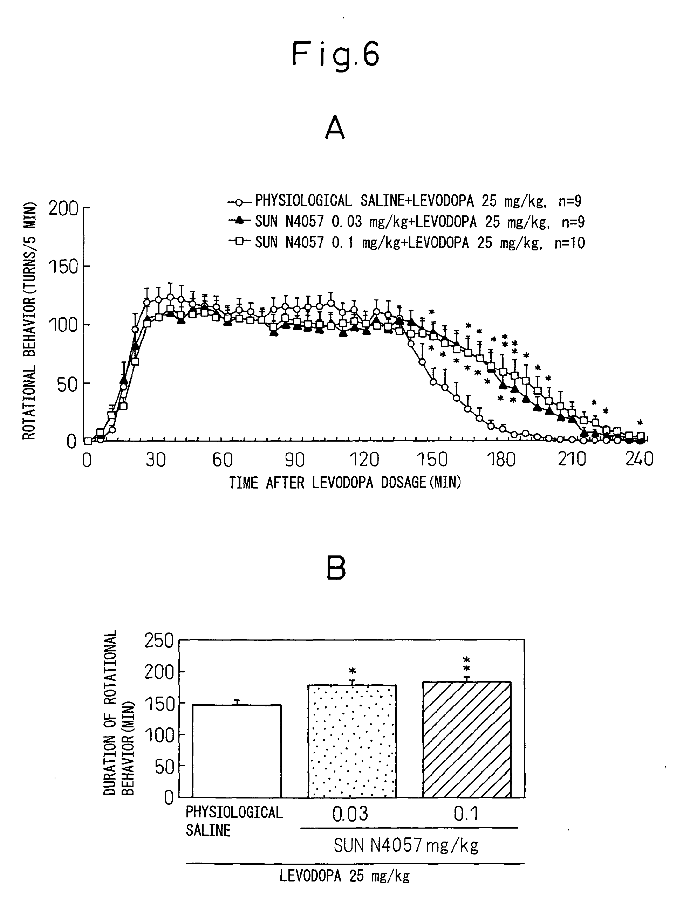

FIG. 6 shows the effects of SUN N4057 repeated subcutaneous administration on rotational behavior of Parkinson's disease model rats (week 7 of repeated doses of levodopa). At week 7 of repeated doses of levodopa (week 2 of SUN N4057 repeated subcutaneous administration), a rotational behavior determining system was used to measure the number of rotations for every 5 minutes after levodopa dosage (A), then the period during which 20% or more of the maximum number of rotations was exhibited in each rat was found and defined as the rotational behavior duration (B). The results are shown by the mean value ± standard error (each group n=9-10). * p<0.05, ** p<0.01: significant difference compared with physiological saline group (A: Dunnett's test, B: Dunnett's test joint type).

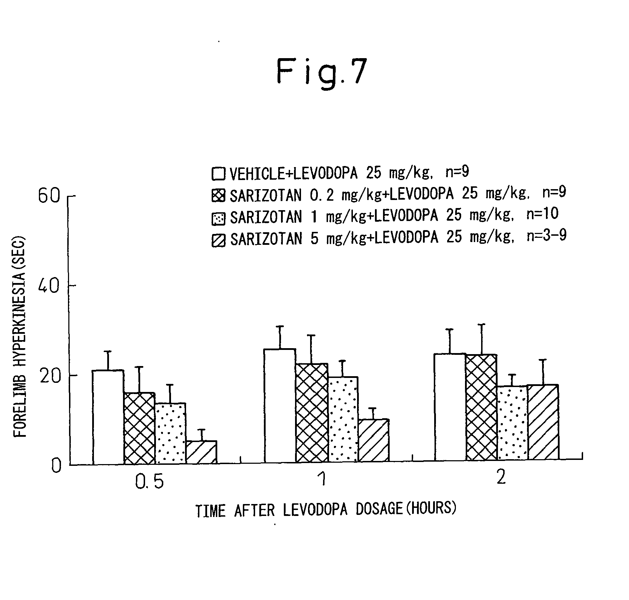

FIG. 7 shows the effects of repeated oral administration of sarizotan on hyperkinesia of the forelimbs of Parkinson's disease model rats (week 7 of repeated doses of levodopa). The period of appearance of hyperkinesia of the forelimbs after levodopa dosage at week 7 of repeated doses of levodopa (week 2 of repeated oral administration of sarizotan) was determined and the results shown by the mean value ± standard error (each group n=9 or 10). Note that in the sarizotan 5 mg/kg pre-administration group, a delay in the start of rotational behavior due to the levodopa dosage was observed. Six out of nine examples 30 minutes after levodopa dosage and three out of nine 1 hour after levodopa dosage did not exhibit rotational behavior, and therefore, hyperkinesia of the forelimbs could not be determined. Therefore, in the sarizotan 5 mg/kg preadministration group, the numbers of examples 30 minutes and 1 hour after levodopa dosage are expressed as respectively n=3 and n=6. There was no significant difference, compared with the solvent group (Dunnett's test).

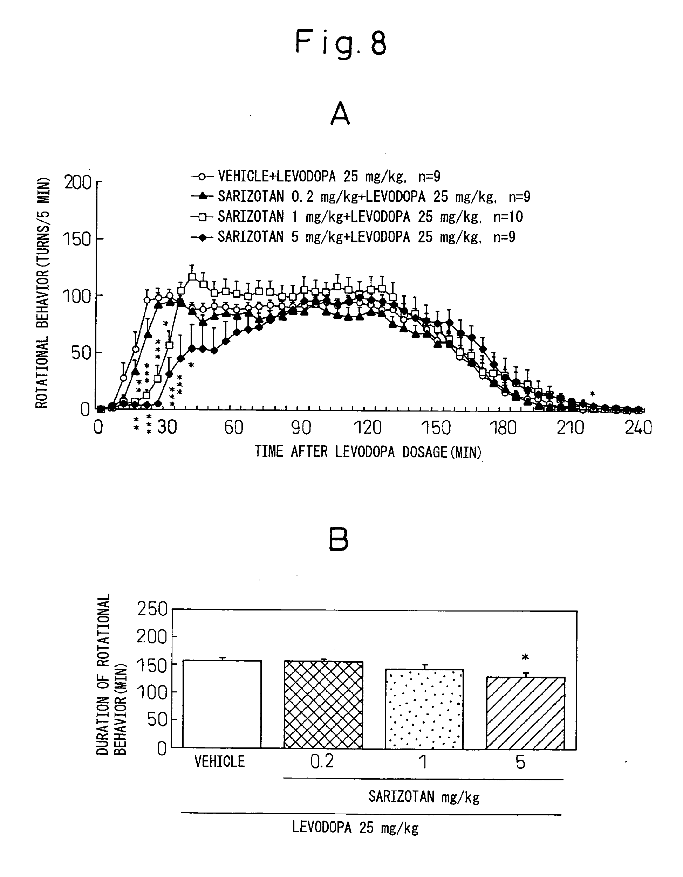

FIG. 8 shows the effects of repeated oral administration of sarizotan on rotational behavior of Parkinson's disease model rats (week 7 of repeated doses of levodopa). At week 7 of repeated doses of levodopa (week 2 of repeated oral administration of sarizotan), a rotational behavior measuring system was used to measure the number of rotations every 5 minutes after levodopa dosage (A), and the period where each rat exhibited 20% or more of the maximum number of rotations was found and defined as the rotational behavior duration (B). The results are shown by the mean value ± standard error (each group n=9-10). * p<0.05, ** p<0.01, *** p<0.001: significant difference compared with solvent group (A: Dunnett's test, B: Dunnett's test joint type).

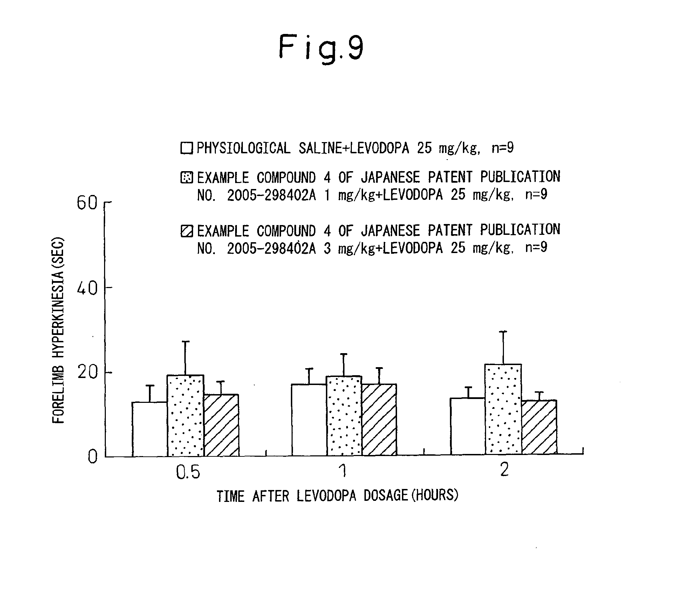

FIG. 9 shows the effects of repeated subcutaneous administration of the Example Compound 4 of Japanese Patent Publication No. 2005-298402A on the hyperkinesia of the forelimbs of Parkinson's disease model rats (week 7 of repeated doses of levodopa). At week 7 of repeated doses of levodopa (week 2 of repeated subcutaneous administration of Example Compound 4 of Japanese Patent Publication No. 2005-298402A), the period of appearance of hyperkinesia of the forelimbs after levodopa dosage was determined and the results shown by the mean value ± standard error (each group n=9). There was no significant difference compared with the physiological saline group (Dunnett's test).

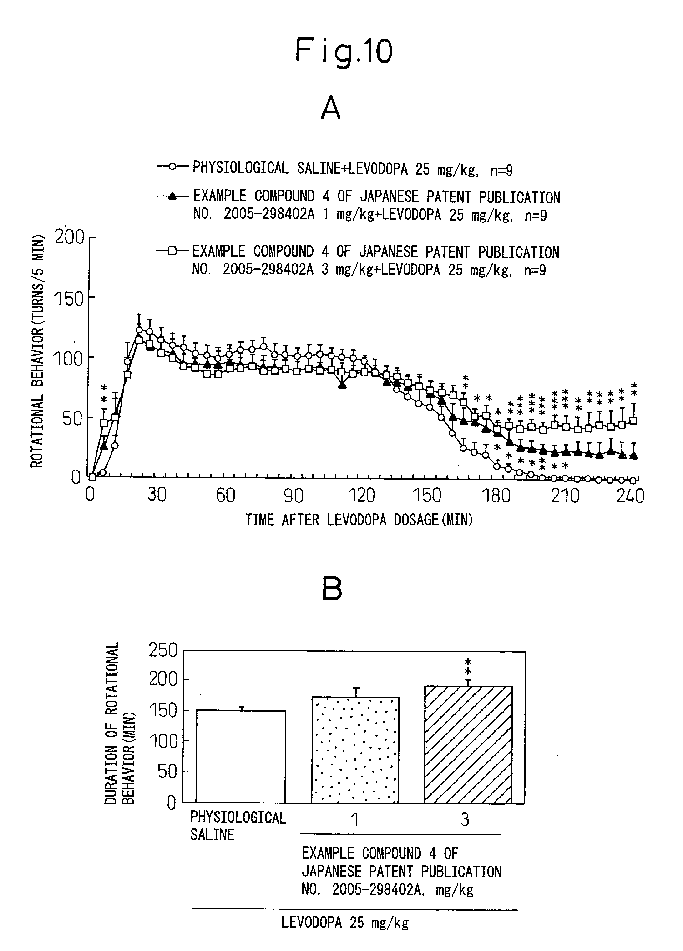

FIG. 10 shows the effects of repeated subcutaneous administration of Example Compound 4 of Japanese Patent Publication No. 2005-298402A on the rotaional behavior of Parkinson's disease model rats (week 7 of repeated doses of levodopa). At week 7 of repeated doses of levodopa (week 2 of repeated subcutaneous administration of Example Compound 4 of Japanese Patent Publication No. 2005-298402A), a rotational behavior determining system was used to determine the number of rotations for every 5 minutes after levodopa dosage (A) then the period in which each rat exhibited 20% or more of the maximum number of rotations was found and defined as the rotational behavior duration (B). The results are shown by the mean value ± standard error (i.e., each group n=9). * p<0.05, ** p<0.01, *** p<0.001: significant difference compared with physiological saline group (A: Dunnett's test, B: Dunnett's test joint type).

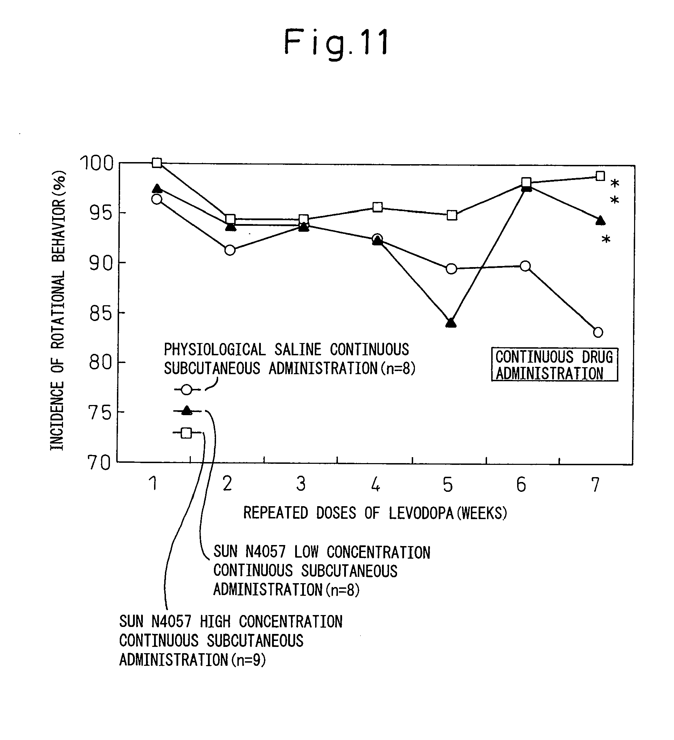

FIG. 11 shows the effects of SUN N4057 sustained subcutaneous administration on the rate of appearance of levodopa dosage-induced rotational behavior using Parkinson's disease model rats. During the repeated doses of levodopa (7 weeks), the presence of any rat rotational behavior about 1 hour after levodopa dosage was recorded. Starting from week 6 of repeated doses of levodopa, an Alzet® osmotic pump (2ML4, volume 2 ml, flow rate 2.5 µl/hr) filled with SUN N4057 or physiological saline was implanted under the skin of each rat. In the subsequent 2 weeks, the presence of any rat rotational behavior about 1 hour after levodopa dosage was recorded. The results are shown by the rate of appearance of rotational behavior for each week of repeated doses of levodopa. The figure shows this by the mean value ± standard error (each group n=8-9). * p<0.05, ** p<0.01: significant difference compared with physiological saline group (Dunnett's test joint type).

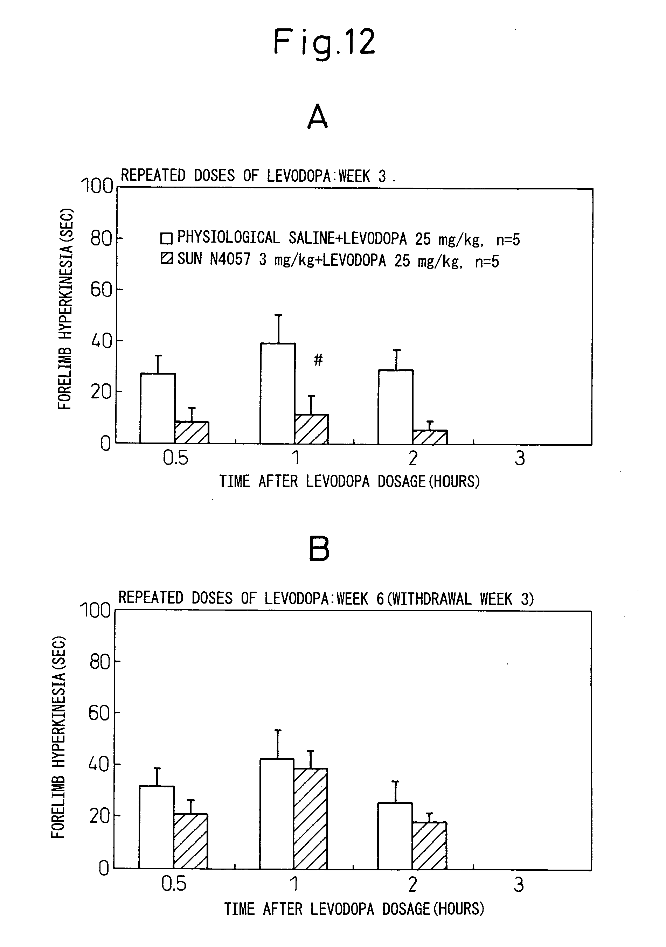

FIG. 12 shows the inhibitory effects of SUN N4057 on the induction of hyperkinesia of the forelimbs induced by repeated doses of levodopa using Parkinson's disease model rats. Along with the start of repeated doses of levodopa, SUN N4057 (3 mg/kg) was intraperitoneally administered for 3 weeks during which the period of appearance of hyperkinesia of the forelimbs was determined (A). Furthermore, after a 3 week withdrawal period (tested substance not administered, only repeated doses of levodopa), the period of appearance of hyperkinesia of the forelimbs in the same rats was determined (B). The results are shown by the mean value ± standard error (each group n=5). # p<0.05: significant difference compared with after withdrawal (t rank test with correspondence).

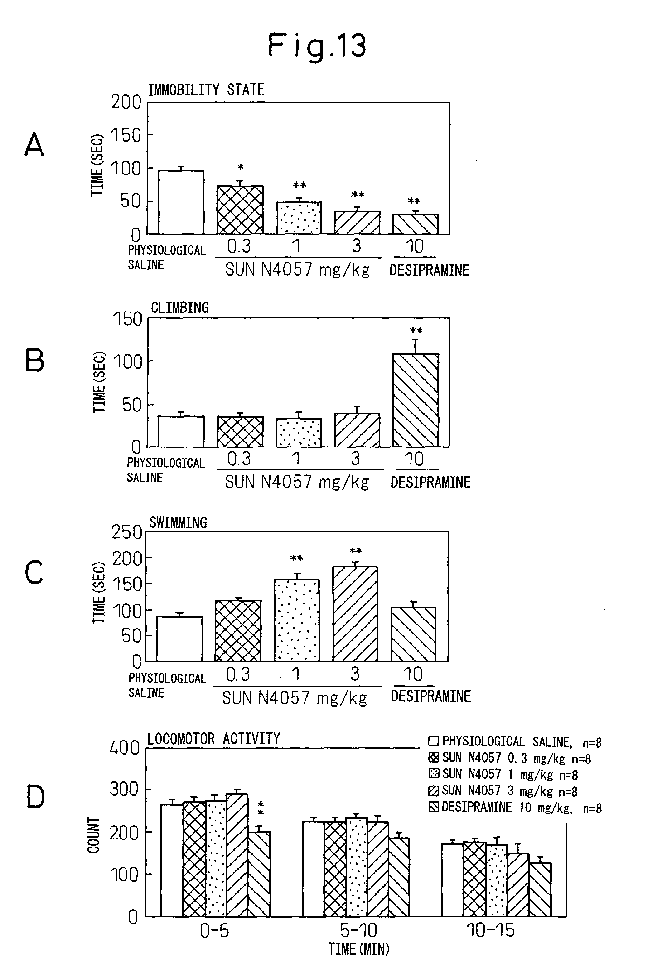

FIG. 13 shows the antidepressive effects of SUN N4057 evaluated using the forced swimming method. The 0-5 minute (5 minutes) immobility period (A), climbing behavior period (B), and swimming behavior period (C) of rats on day 2 of swimming were determined. The amount of spontaneous movement (D) was determined using a determining system set in a blacked out soundproof box and determining the amount of spontaneous movement of the rats in minutes 0-15 (15 minutes). The results for every 5 minutes are shown. The results are shown by the mean value ± standard error (each group n=8). * p<0.05, ** p<0.01: significant difference compared with physiological saline group (Dunnett's test).

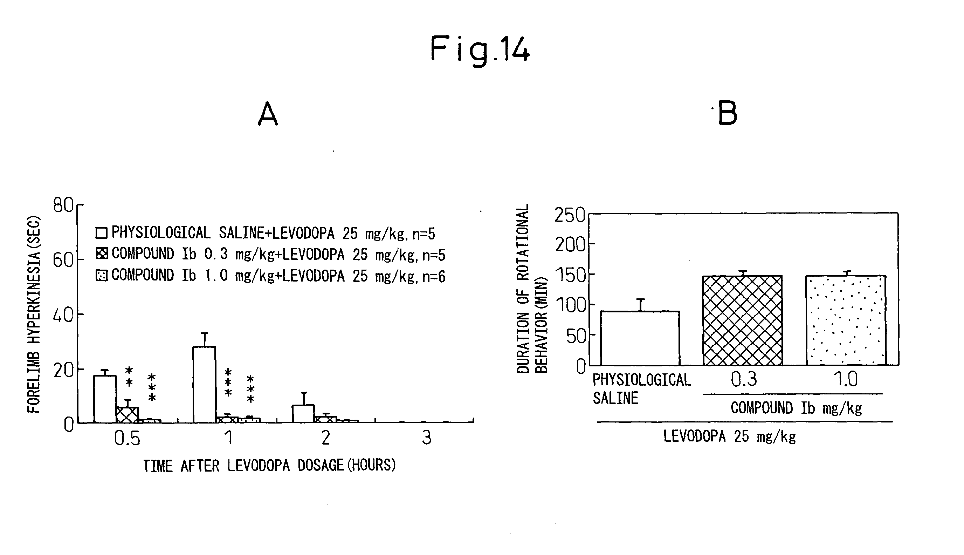

FIG. 14 shows the effects of repeated intraperitoneal administration of the compound Ib on hyperkinesia of the forelimbs and rotational behavior duration of Parkinson's disease model rat (week 7 of repeated doses of levodopa). The period of appearance of hyperkinesia of the forelimbs after levodopa dosage at week 7 of repeated doses of levodopa (week 2 of repeated intraperitoneal administration of the compound Ib) was determined (A), the number of rotations for every 5 minutes after levodopa dosage was determined, and the period during which each rat exhibited 20% or more of the maximum number of rotations was found and defined as the rotational behavior duration (B). The results are shown by the mean value ± standard error (each group n=5-6). * p<0.05, ** p<0.01, *** p<0.001: significant difference compared with physiological saline group (A: Dunnett's test, B: Dunnett's test joint type).

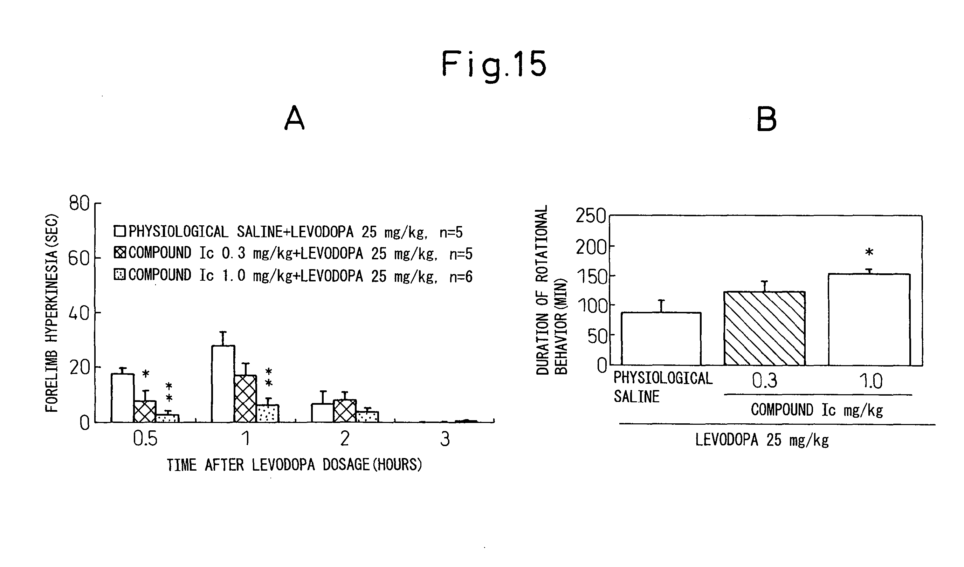

FIG. 15 shows the effect of repeated intraperitoneal administration of the compound Ic on hyperkinesia of the forelimbs and rotational behavior duration of Parkinson's disease model rats (week 7 of repeated doses of levodopa). The period of appearance of hyperkinesia of the forelimbs after levodopa dosage at week 7 of repeated doses of levodopa (week 2 of repeated intraperitoneal administration of the compound Ic) was determined (A), the number of rotations for every 5 minutes after levodopa dosage was determined, and the period during which each rat exhibited 20% or more of the maximum number of rotations was found and defined as the rotational behavior duration (B). The results are shown by the mean value ± standard error (each group n=5-6). * p<0.05, ** p<0.01, *** p<0.001: significant difference compared with physiological saline group (A: Dunnett's test, B: Dunnett's test joint type).

### BEST MODE FOR CARRYING OUT THE INVENTION

The singular used in the Description and the attached Claims (that is, "a", "an", and "the") should be understood as including the plural except in cases where it is otherwise clearly not so from the context.

The inventors first engaged in in-depth research on the mechanism of occurrence of dyskinesia, the wearing off phenomenon, and the on-off phenomenon accompanying levodopa therapy of Parkinson's disease and the relation with the actions of the dopamine nervous system and serotonin nervous system.

Further, serotonin 1A receptor agonists reportedly may alleviate motor complications, but regarding the relationship between their actions and the therapeutic effect, the inventors assume that serotonin 1A receptor agonists inhibit the sudden rise of striatal dopamine concentration after levodopa dosage so as to inhibit dyskinesia and keep the striatal dopamine concentration within the safe treatment region for a long period of time so as to also mitigate the wearing off phenomenon and, further, inhibit the progressive degeneration of nigrostriatal dopamine nerves by the nerve cell protective action. Further, serotonin 1A receptor agonists are classified by form of action into full agonists and partial agonists (i.e., partially activated weaker in action than full agonists), but in the case of using a high dosage, full agonists act too powerfully and are liable to have a detrimental effect on the treatment of Parkinson's disease by levodopa. Further, in clinical studies, setting the suitable dosage becomes difficult. With a compound having a partial agonist action, however, due to the strength of its action, these concerns can be eliminated. Therefore, this is considered preferable.

Further, the inventors considered that it is important not to have an antagonist action against dopamine D2 receptors liable to cause weakening of the therapeutic effect of levodopa on Parkinson's disease. Further, having an agonist action against dopamine D3 receptors acts advantageously for nerve cell protection, and therefore, is considered preferable.

Thus, the inventors searched for a drug having a serotonin 1A receptor agonist (i.e., partial agonist) action, not having an antagonist action against dopamine D2 receptors, and having an agonist action against dopamine D3 receptors. As a result, the inventors found that a compound having the formula (I): 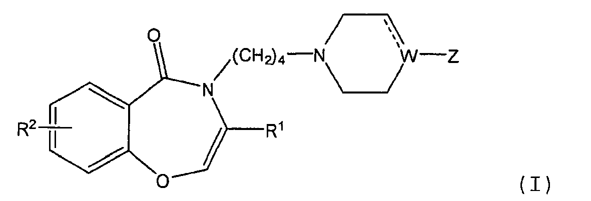 wherein R¹ indicates a hydrogen atom, chlorine atom, or C₁ to C₄ alkyl group, R² indicates a hydrogen atom, halogen atom, methyl group or methoxy group, W indicates a nitrogen atom, CH or carbon atom, the dotted line indicates the absence of a bond when W is a nitrogen atom or CH and indicates the presence of a bond when W is a carbon atom, Z indicates a phenyl group, pyridyl group or pyrimidinyl group, which is unsubstituted or substituted with 1 to 3 substituent groups selected from the group consisting of a methyl group, methoxy group and halogen atom or a pharmacologically acceptable salt thereof or their hydrates (hereinafter referred to as "the present compound") has desirable properties.

The present compound is a compound described in International Patent Publication No. WO 96/24594. International Patent Publication No. WO 96/24594 discloses that this has a strong affinity with serotonin 1A receptors, exhibits a weak affinity with dopamine D2 receptors, exhibits an antianxiety action based on the indicator of anticonflict action, has suppressive activity in cerebral infraction and other brain protective actions in ischemic brain disorders in the transitory right middle cerebral artery occlusion (MCAO) model, and is useful for the treatment of anxiety neurosis, phobia, obsessive-compulsive disorder, schizophrenia, post-traumatic stress disorder, depressive neurosis, psychosomatic disorders, and other psychiatric and neurological disorders, eating disorders, menopausal disorders, infantile autism, and other disorders and other disorders involving the cerebral circulatory system accompanied with vomiting or cerebral occlusion and cerebral hemorrhaging. Further, it discloses that, due to the difference in affinity with serotonin 1A receptors and dopamine D2 receptors, it is useful as an antidepressive and antianxiety drug with few side effects. However, International Patent Publication No. WO 96/24594 does not clarify if the present invention compound is a full agonist with respect to serotonin 1A receptors or a partial agonist and if it is an agonist or an antagonist with respect to dopamine D2 receptors. Further, the action against dopamine D3 receptors is not described at all. There is no description at all regarding therapeutic effect on motor complications in levodopa therapy of Parkinson's disease, in particular advanced stage Parkinson's disease, or accompanying psychiatric symptoms.

The inventors studied the therapeutic effect on motor complications and accompanying psychiatric symptoms of advanced stage Parkinson's disease patients by preparing an animal model exhibiting changes in behavior resembling motor complications and studying the effects of the compound, whereupon they found that it has effects alleviating and inhibiting the appearance of changes in behavior like motor complications associated with repeated doses of levodopa. Furthermore, the inventors found that this is also effective against anxiety and other psychiatric symptoms and thereby completed the present invention.

That is, the present invention provides the following:
(1) A drug for alleviating motor complications associated with a treatment with levodopa for Parkinson's disease containing a compound having the formula (I) or a pharmacologically acceptable salt thereof or their hydrates.
(2) A drug according to (1), wherein, in the formula (I), R¹ indicates a chlorine atom, R² indicates a hydrogen atom, W indicates a carbon atom, the dotted line indicates the presence of a bond and Z indicates a pyridyl group or pyrimidinyl group which is unsubstituted or substituted with a methyl group.
(3) A drug according to (1), wherein the compound having the formula (I) is 3-chloro-4,5-dihydro-4-{4-[4-(2-pyridyl)-1,2,3,6-tetrahydropyridin-1-yl]butyl}-1,4-benzoxazepin-5-one.
(4) A drug according to any one of (1) to (3) above, which reduces dyskinesia in motor complications associated with a treatment with levodopa for Parkinson's disease.
(5) A drug according to any one of (1) to (3), which reduces the off time in levodopa therapy of Parkinson's disease.
(6) A drug according to any one of (1) to (3), which prolongs the duration of pharmacological effect of levodopa (i.e., on time) not accompanied with dyskinesia in levodopa therapy of Parkinson's disease.
(7) A drug according to any one of (1) to (3), which reduces the frequency of appearance of the on-off phenomenon in motor complications accompanying levodopa therapy for Parkinson's disease.
(8) A drug according to any one of (1) to (3) which suppresses the increase in the amount of levodopa required for the treatment accompanying the advance of symptoms in levodopa therapy of Parkinson's disease.
(9) A drug according to any one of (1) to (3), which reduces the number of dosages of levodopa per day required for treatment in levodopa therapy of Parkinson's disease.
(10) A drug inhibiting or delaying the advance of symptoms of Parkinson's disease comprising a compound having the formula (I) or a pharmacologically acceptable salt thereof or their hydrates.
(11) A drug according to (10), wherein, in the formula (I), R¹ indicates a chlorine atom, R² indicates a hydrogen atom, W indicates a carbon atom, the dotted line indicates the presence of a bond and Z indicates a pyridyl group or pyrimidinyl group which is unsubstituted or substituted with a methyl group.
(12) A drug according to (10), wherein the compound having to formula (I) is 3-chloro-4,5-dihydro-4-{4-[4-(2-pyridyl)-1,2,3,6-tetrahydropyridin-1-yl]butyl}-1,4-benzoxazepin-5-one.
(13) A drug for delaying the onset of motor complications associated with a treatment of levodopa for Parkinson's disease comprising a compound having the formula (I) or a pharmacologically acceptable salt thereof or their hydrates.
(14) A drug according to (13), wherein in the formula (I), R¹ indicates a chlorine atom, R² indicates a hydrogen atom, W indicates a carbon atom, the dotted line indicates the presence of a bond and Z indicates a pyridyl group or pyrimidinyl group which is unsubstituted or substituted with a methyl group.
(15) A drug according to (13), wherein the compound having the formula (I) is 3-chloro-4,5-dihydro-4-{4-[4-(2-pyridyl)-1,2,3,6-tetrahydropyridin-1-yl]butyl}-1,4-benzoxazepin-5-one.
(16) A drug for alleviating a psychiatric symptom accompanying advanced stage Parkinson's disease comprising a compound having the formula (I) or a pharmacologically acceptable salt thereof or their hydrates.
(17) A drug according to (16), wherein in the formula (I), R¹ indicates a chlorine atom, R² indicates a hydrogen atom, W indicates a carbon atom, the dotted line indicates the presence of a bond and Z indicates a pyridyl group or pyrimidinyl group which is unsubstituted or substituted with a methyl group.
(18) A drug according to (16), wherein the compound having the formula (I) is 3-chloro-4,5-dihydro-4-{4-[4-(2-pyridyl)-1,2,3,6-tetrahydropyridin-1-yl]butyl}-1,4-benzoxazepin-5-one.
(19) A drug according to any one of (16) to (18),
   wherein the psychiatric symptom accompanying advanced stage Parkinson's disease is depression, anxiety, or hallucinations.
(20) A drug according to any one of (1) to (19), which is used, in combination with at least one drug selected from peripheral dopa-decarboxylase inhibitors, monoamine oxidase type B inhibitors, and catechol-o-methyl transferase inhibitors (note that here, "used in combination" includes simultaneous administration of both drugs, administration as a combination drug of the two, and further administration of the two drugs separately and independently in time).
(21) A pharmaceutical composition for treatment of Parkinson's disease comprising (a) a compound having the formula (I) or a pharmacologically acceptable salt thereof or their hydrates and (b) levodopa.
(22) A pharmaceutical composition for treatment of Parkinson's disease comprising (a) a compound having the formula (I) or a pharmacologically acceptable salt thereof or their hydrates, (b) levodopa, and (c) one or more drugs selected from peripheral dopa-decarboxylase inhibitors, monoamine oxidase type B inhibitors, and catechol-o-methyl transferase inhibitors.
(23) A composition according to (21) or (22) wherein, in the formula (I), R¹ indicates a chlorine atom, R² indicates a hydrogen atom, W indicates a carbon atom, the dotted line indicates the presence of a bond, and Z indicates a pyridyl group or pyrimidinyl group which is unsubstituted or substituted by a methyl group.
(24) A composition according to (21) or (22) wherein the compound having the formula (I) is 3-chloro-4,5-dihydro-4-{4-[4-(2-pyridyl)-1,2,3,6-tetrahydro-pyridin-1-yl]butyl}-1,4-benzoxazepin-5-one.

The present compound has a serotonin 1A receptor agonist (i.e., partial agonist) action and does not have an antagonist action against dopamine D2 receptors. From the later explained test results of advanced stage Parkinson's disease model rats, it was learned that an action for alleviating motor complications associated with a treatment with repeated doses of levodopa and an action inhibiting the appearance of motor complications are exhibited and the action of levodopa is not weakened. Further, it was learned that the present compound can inhibit the sudden rise in dopamine concentration due to levodopa in Parkinson's disease patients and keep the dopamine concentration in the safe treatment area for a long time, increases the rate of utilization of levodopa, inhibits the increase in the amount of levodopa required for a treatment along with the advance of Parkinson's disease symptoms, and reduces the number of dosages per day of levodopa required for treatment or inhibits the increase in the number of dosages. Further, it became clear that the present compound also exhibits effects against accompanying psychiatric symptoms in advanced stage Parkinson's disease patients. Furthermore, it became clear that the present compound has not only a serotonin 1A receptor agonist (partial agonist) action, but also an agonist action against dopamine D3 receptors and was learned that it also has an effect inhibiting or delaying the advance of symptoms of the progressive neurodegeneration disorder of Parkinson's disease. Further, in clinical tests studying the efficacy and safety as a drug for treatment of motor complications in undergoing Parkinson's disease patients levodopa therapy, the effects of the present invention compound were shown. Therefore, the present invention enabled the provision of a new drug for treatment of Parkinson's disease.

In the present compound having the formula (I), as a preferable example of the group R¹, a chlorine atom may be mentioned, while as a preferable example of the group R², a hydrogen atom may be mentioned. As a preferable example of W, a carbon atom may be mentioned. At this time, the dotted line indicates the presence of a bond. Further, as a preferable example of the group Z, a pyridyl group or pyrimidinyl group which is unsubstituted or substituted with a methyl group may be mentioned. More preferably, a pyridyl group may be mentioned.

As specific preferable embodiments of the compound having the formula (I), 3-chloro-4,5-dihydro-4-{4-[4-(2-pyridyl)-1,2,3,6-tetrahydropyridin-1-yl]butyl}-1,4-benzoxazepin-5-one, 3-chloro-4,5-dihydro-4-{4-[4-(2-pyrimidinyl)-1,2,3,6-tetrahydropyridin-1-yl]butyl}-1,4-benzoxazepin-5-one and 3-chloro-4,5-dihydro-4-(4-{4-[(4-methyl)-2-pyrimidinyl]-1,2,3,6-tetrahydropyridin-1-yl}butyl)-1,4-benzoxazepin-5-one may be mentioned. Among these, 3-chloro-4,5-dihydro-4-{4-[4-(2-pyridyl)-1,2,3,6-tetrahydropyridin-1-yl]butyl}-1,4-benzoxazepin-5-one (a compound of the following formula (II)) is particularly preferable.

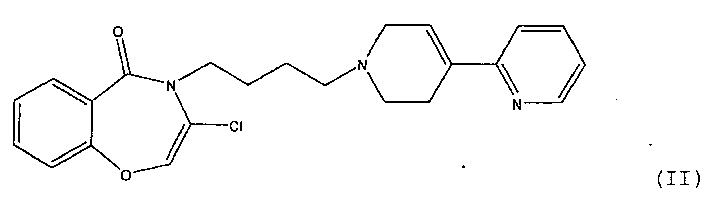

As the pharmacologically acceptable salts in the present invention, hydrochlorides, nitrates, sulfates, hydrobromides, phosphates, and other inorganic acid salts and also methane sulfonates, acetates, oxalates, succinates, malonates, tartarates, maleates, fumarates, lactates, citrates and other organic acid salts may be mentioned. Among these, hydrochlorides and fumarates are preferred.

As a particularly preferable specific example of the present compound, 3-chloro-4,5-dihydro-4-{4-[4-(2-pyridyl)-1,2,3,6-tetrahydropyridin-1-yl]butyl}-1,4-benzoxazepin-5-one·dihydrochloride-dihydrate (compound Ia), 3-chloro-4,5-dihydro-4-{4-[4-(2-pyrimidinyl)-1,2,3,6-tetrahydropyridin-1-yl]butyl}-1,4-benzoxazepin-5-one hydrochloride (compound Ib) and 3-chloro-4,5-dihydro-4-(4-{4-[(4-methyl)-2-pyrimidinyl]-1,2,3,6-tetrahydropyridin-1-yl}butyl)-1,4-benzoxazepin-5-one fumarate (compound Ic) may be mentioned. In particular, the compound Ia having the following formula (III) (hereinafter referred to as "SUN N4057") is optimal for the alleviation of motor complications associated with a treatment with levodopa for Parkinson's disease or psychiatric symptoms accompanying advanced stage Parkinson's disease.

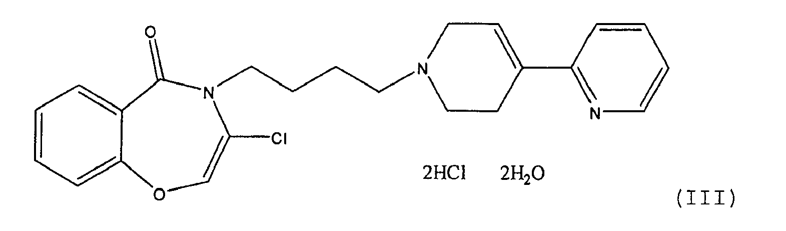

The compound having the formula (I) of the present invention or a pharmacologically acceptable salt thereof or their hydrates can be produced by known methods such as the methods described in International Patent Publication No. WO 96/24594, Japanese Patent Publication No. 2000-516640A, Japanese Patent Publication No. 2001-507373A, Kamei K. et al., "A practical synthetic method for vinyl chlorides and vinyl bromides from ketones via the corresponding vinyl phosphate intermediates", Tetrahedron Letters, 2005, 46, p. 229-232 and existing reports (above-mentioned Kamei K. et al., "New 5-HT1A Receptor Agonists Possessing 1,4-Benzoxazepine Scaffold Exhibit Highly Potent Anti-Ischemic Effects", Bioorganic & Medicinal Chemistry Letters, 2001, 11, p. 595-598, Kamei K. et al., "New piperidinyl- and 1,2,3,6-tetrahydropyridinyl-pyrimidine derivatives as selective 5-HT1A receptor agonists with highly potent anti-ischemic effects", Bioorganic & Medicinal Chemistry Letters, 2005, 15, p. 2990-2993, Kamei K. et al., "Synthesis, SAR studies, and evaluation of 1,4-benzoxazepine derivatives as selective 5-HT1A receptor agonists with neuroprotective effect: Discovery of Piclozotan", Bioorganic & Medicinal Chemistry, 2006, 14, p. 1978-1992).

The present invention provides a drug for alleviating motor complications associated with a treatment with levodopa for Parkinson's disease. In the present invention, "alleviating motor complications" means mitigating or inhibiting motor symptoms becoming a problem in the treatment observed in advanced stage Parkinson's disease patients, that is, motor complications (involuntary motion associated with a treatment with levodopa, that is, dyskinesia, and wearing off phenomenon or on-off phenomenon and other daily fluctuations of symptoms or motor fluctuations). Motor complications can be judged by the Unified Parkinson's Disease Rating Scale (UPDRS), Part 4: Complications of Therapy. Alleviation of motor complications includes reduction of dyskinesia, reduction of the off time, prolongation of the duration of pharmacological effect of levodopa (on time) not accompanied with dyskinesia and reduction of the frequency of occurrence of the on-off phenomenon. Further, inhibition of the increase in the amount of levodopa required for treatment along with the advance of symptoms and reduction of the number of times of dosage per day required in treatment with levodopa are also included.

In the present invention, "reducing dyskinesia" means reducing the duration of involuntary motion accompanying levodopa therapy seen in the mouth, tongue, face, limbs, and body trunk, that is, dyskinesia (percentage during waking period of patients) or reducing the degree of the disorders. Dyskinesia can be judged by the Unified Parkinson's Disease Rating Scale (UPDRS) Part 4: Complications of Therapy, Part A, in particular items 32 and 33.

In the present invention, "reducing the off time" means reducing the percentage of the period in which the effects of the levodopa are insufficient and the symptoms of Parkinson's disease (tremors, rigidity, akinesia, and postural instability) appear, that is, the off time (percentage during waking period of patients). The off time can be judged by the Unified Parkinson's Disease Rating Scale (UPDRS), Part 4: Complications of Therapy, Part B, in particular item 39.

In the present invention, "prolonging the duration of the pharmacological effect of levodopa (on time) not accompanied with dyskinesia" means increasing the percentage of the period in which Parkinson's disease patients do not experience involuntary motion associated with a treatment with levodopa seen in the mouth, tongue, face, limbs, and body trunk, that is, dyskinesia, and taking levodopa results in symptoms of Parkinson's disease (tremors, rigidity, akinesia, and postural instability) being relatively suppressed (on time) (percentage during waking period of patients). Symptoms of Parkinson's disease can be judged by the Unified Parkinson's Disease Rating Scale (UPDRS). Further, Parkinson's disease patients can self assess the presence or absence of effects after taking levodopa, that is, the on time or the off time.

In the present invention, "reducing the frequency of appearance of the on-off phenomenon" means reducing the number of times of appearance of the phenomenon where, regardless of the period of taking levodopa (elapse of time after taking it), the symptoms are alleviated (on) or suddenly deteriorate (off), that is, the on-off phenomenon. It is reported that the on-off phenomenon sometimes repeats several times a day and is accompanied with dyskinesia in the on periods ("Japanese Society of Neurology Treatment Guidelines: Parkinson's Disease Treatment Guideline 2002", Ad Hoc Committee, Rinsho Shinkeigaku (Clinical Neurology), 2002, 42, p. 430-94).

The present invention further provides "levodopa treatment of Parkinson's disease, wherein the increase of the amount of levodopa required for treatment along with the advance of the symptoms is suppressed". Further, the present invention provides "the levodopa treatment of Parkinson's disease wherein the number of dosages of levodopa per day required for treatment is reduced". Levodopa is the drug exhibiting the most powerful action in alleviating symptoms in drugs for Parkinson's disease and forms the core of drug therapy of Parkinson's disease. However, the half life of the drug is short (0.5 to 1 hour). As the Parkinson's disease advances, the duration of action of the levodopa is reduced and Parkinson's disease patients have to be administered higher dosages of levodopa or administered levodopa more frequently. Examples are seen where at the start of levodopa therapy, the drug was administered 2 to 3 times a day, but had to be increased up to 5 to 8 times. This becomes a problem in the quality of life of patients. Note that usually levodopa is administered in peripheral dopa-decarboxylase inhibitor combinations (i.e., levodopa-carbidopa combinations or levodopa-benserazide combinations in daily amounts of use of usually 300 to 1200 mg). A peripheral dopa-decarboxylase inhibitor (i.e., carbidopa or benserazide) blocks the metabolism of levodopa to dopamine, but does not pass through the blood-brain barrier, and therefore, does not block the metabolism to dopamine in the brain. Since the metabolism to dopamine at the peripheral organs is inhibited, the amount of levodopa required is decreased and the side effects in the digestive system are mitigated. For this reason, introduction of treatment becomes easy, but, on the other hand, the frequency of dyskinesia, believed to be caused by the sudden increase of the striatal dopamine concentration, increases ("Japanese Society of Neurology Treatment Guidelines: Parkinson's Disease Treatment Guideline 2002", Ad Hoc Committee, Rinsho Shinkeigaku (Clinical Neurology), 2002, 42, p. 430-94). The present compound can inhibit the sudden increase in the striatal dopamine concentration after levodopa dosage, based on its serotonin 1A receptor agonist (partial agonist) action and thereby inhibit the motor complication dyskinesia. Furthermore, by keeping the striatal dopamine concentration in the safe treatment range for a longer period, the rate of utilization of levodopa can be improved. It is also possible to inhibit the increase in the amount of levodopa required for treatment accompanying the advance of Parkinson's disease symptoms or reduce the number of dosages of levodopa per day required for treatment or suppress the increase in number of times of dosage. In the present invention, "reducing the number of dosages of levodopa per day required for treatment" means both inhibiting the increase in number of dosages of levodopa required along with the advance of symptoms and decreasing the already required number of dosages of levodopa.

Further, the present invention provides a drug inhibiting or delaying the advance of symptoms of Parkinson's disease. In the present invention, "inhibiting or delaying the advance of symptoms of Parkinson's disease" means inhibiting neurodegeneration or delaying the speed of neurodegeneration from normal based on the protective and reparative action on nigrostriatal dopamine nerves for Parkinson's disease - a progressive degenerative disorder of the nigrostriatal dopamine nerves where symptoms deteriorate along with the advance of neurodegeneration. The advance of symptoms of Parkinson's disease (severity) can be judged by the Hoehn & Yahr Staging and the Unified Parkinson's Disease Rating Scale (UPDRS). If it were possible to delay or repair the degeneration of nigrostriatal dopamine nerves in Parkinson's disease, a great therapeutic effect could be expected. However, there is still no drug succeeding in the treatment of Parkinson's disease by retarding or repairing neurodegeneration. In actuality, the Japanese Society of Neurology Treatment Guidelines for Parkinson's disease ("Japanese Society of Neurology Treatment Guidelines: Parkinson's Disease Treatment Guideline 2002", Ad Hoc Committee, Rinsho Shinkeigaku (Clinical Neurology), 2002, 42, p. 430-94) also states that if there were a drug for Parkinson's disease having a clear nerve cell protective effect, it should be immediately used after diagnosis. The present compound has an agonist action against dopamine D3 receptors and has a nerve cell protective effect, and therefore, can inhibit or delay the advance of symptoms of Parkinson's disease.

The present invention further provides a drug delaying the onset of motor complications associated with a treatment with levodopa for Parkinson's disease. In the present invention, "delaying the onset of motor complications associated with a treatment with levodopa" means delaying the timing of the onset of dyskinesia, the wearing off phenomenon, on-off phenomenon and other motor complications in Parkinson's disease patients along with long term administration of levodopa. In Parkinson's disease, the delay of the onset of motor complications is a major unmet need in treatment of Parkinson's disease along with drugs having a protective and reparative action on nigrostriatal dopamine nerves. There is reportedly a broad range in the onset frequency of motor complications due to long-term administration of levodopa in Parkinson's disease patients, but if the treatment period extends to 4 to 6 years or more, it is reported that about 40% of Parkinson's disease patients experience the onset of complications (Ahlskog J.E. et al., "Frequency of levodopa-related dyskinesias and motor fluctuations as estimated from the cumulative literature", Movement Disorders, 2001, 16, p. 448-458). The Japanese Society of Neurology Treatment Guidelines for Parkinson's disease ("Japanese Society of Neurology Treatment Guidelines: Parkinson's Disease Treatment Guideline 2002", Ad Hoc Committee, Rinsho Shinkeigaku (Clinical Neurology), 2002, 42, p. 430-94) also states that about every year after the start of levodopa therapy, another 10% of patients experience onset of complications and that after 5 years 50% of patients suffer from daily fluctuations in symptoms. Furthermore, the Japanese Society of Neurology Treatment Guidelines for Parkinson's disease recommends, for all but some of the patients (70 to 75 years old or older patients and patients having cognitive function disorders), the start of treatment at the early stage of onset of Parkinson's disease not by levodopa, but by a dopamine D2 receptor agonist and states that the biggest reason for this is to delay the onset of daily fluctuations in motor symptoms and dyskinesia. Further, it is considered important not to use more than the necessary amount of levodopa so as to delay even a bit the daily fluctuations expected in the future. The present compound can inhibit the sudden increase in striatal dopamine concentration after levodopa dosage and keep the striatal dopamine concentration in the safe treatment range longer based on the serotonin 1A receptor agonist (partial agonist) action. Therefore, by administering the present compound simultaneously with the start of levodopa therapy, it is possible to increase the rate of utilization of levodopa and maintain the amount of levodopa required for treatment at a low dosage over a long period and possible to delay the onset of motor complications.

The present invention further provides a drug for alleviating the psychiatric symptoms accompanying advanced stage Parkinson's disease. In the present invention, "alleviating psychiatric symptoms" means mitigating or inhibiting the psychiatric symptoms (depression, anxiety, hallucinations, etc.) in the non-motor symptoms becoming problems in treatment observed in advanced stage Parkinson's disease patients. As explained above, in Parkinson's disease, not only motor symptoms, but also non-motor symptoms become factors impairing the quality of life of patients due to long-term treatment of Parkinson's disease and the prolongation of the lives of the patients. Treatment of these, it is pointed out, is important. Psychiatric symptoms can be judged by the Unified Parkinson's Disease Rating Scale (UPDRS) Part 1: Mentation, Behavior and Mood. The depression and anxiety in Parkinson's disease are considered highly likely to be due to the motor disorders of patients. As treatment of psychiatric symptoms in advanced stage Parkinson's disease, depression is sometimes treated with antidepressants, but tricyclic antidepressants sometimes cause deterioration of Parkinson's disease. Therefore, recently, selective serotonin reuptake inhibitors (SSRI) are becoming the mainstream. However, there are also reports of deterioration of Parkinson's disease due to use of SSRI. Further, use of an SSRI and the monoamine oxidase B Selegiline together is likely to cause serotonin syndromes and is avoided in Japan ("Japanese Society of Neurology Treatment Guidelines: Parkinson's Disease Treatment Guideline 2002", Ad Hoc Committee, Rinsho Shinkeigaku (Clinical Neurology), 2002, 42, p. 430-94). Further, when hallucinations appears in advanced stage Parkinson's disease, in principle the anti-Parkinson's disease drugs which had been used for treatment up to then are successively stopped, the prescription is simplified, and finally only levodopa is used for treatment. When decrease in the amount of levodopa is difficult, it is prescribed that a small amount of an atypical antipsychotic be used ("Japanese Society of Neurology Treatment Guideline: Parkinson's Disease Treatment Guideline 2002", Ad Hoc Committee, Rinsho Shinkeigaku (Clinical Neurology), 2002, 42, p. 430-94), but side effects of atypical antipsychotics have also been pointed out and control of Parkinson's disease symptoms becomes difficult, and therefore, there is a large effect on the quality of life of patients. The pathogenesis of hallucinations in Parkinson's disease patients is not clear, but it is hypothesized that this is because the dopamine receptors of the cortex are continually stimulated unphysiologically by excessive dopamine. Further, the possibility of abnormalities in the serotonin function being involved in the psychiatric symptoms has been pointed out (Melamed E. et al., "Involvement of serotonin in clinical features of Parkinson's disease and complications of L-DOPA therapy", Advances in Neurology, 1996, 69, p. 545-550). In actuality, it is reported that drugs inhibiting brain serotonin release alleviate psychiatric symptoms of advanced stage Parkinson's disease (Zoldan J. et al., "Psychosis in advanced Parkinson's disease: Treatment with ondansetron, a 5-HT3 receptor antagonist", Neurology, 1995, 45, p. 1305-1308). Furthermore, as behavioral disorders of Parkinson's disease patients, in addition to depression, anxiety and panic attacks have also been reported. These are sometimes seen synchronously with the off time and are taken note of in considering the disease conditions and treatment (Kashiwabara Kenichi, "Behavioral impairments", Nippon Rinsho (Japanese Journal of Clinical Medicine), 2004, 62, p. 1675-1678). The present invention compound has the action of not weakening the therapeutic effect of levodopa, but rather increasing its rate of utilization. By inhibiting the excess stimulation of dopamine and inhibiting brain serotonin release, it is possible to alleviate psychiatric symptoms, without weakening the levodopa therapeutic effect in advanced stage Parkinson's disease patients.

The present invention will now be explained in detail along with Examples, but the scope of the present invention is not limited to these Examples.

SUN N4057, as shown in Example 1, exhibits a strong binding affinity of a Ki value of 0.0249 nM with human serotonin 1A receptors, but only exhibits weak binding affinities of Ki values of 161 nM and 162 nM with human dopamine D2L receptors and dopamine D2S receptors. Note that dopamine D2 receptors are classified into two types by the presence or absence of 29 amino acid residues at the third loops in the cells. These are called "D2 Long" (described as "D2L") and "D2 Short" (described as "D2S"). The D2L are present in the post-synapse membranes, while the D2S are present in the pre-synapse membranes. Furthermore, as shown in Example 2, as the functions, SUN N4057 has agonist actions against human serotonin 1A receptors and human dopamine D3 receptors (EC50 values of 2.41 nM and 2.12 nM, respectively), but does not have antagonist actions against human dopamine D2L receptors and D2S receptors (EC50 values: >500 nM). In the treatment of Parkinson's disease, levodopa and dopamine D2 receptor agonists are used as treatment drugs. The dopamine D2 receptor antagonist action is liable to cause weakening in the Parkinson's disease therapeutic effect. Therefore, SUN N4057 not having any dopamine D2 receptor antagonist action promises the effect based on the serotonin 1A receptor and dopamine D3 receptor agonists, without reducing the therapeutic effect of levodopa on Parkinson's disease.

Furthermore, serotonin 1A receptor agonists are classified from their form of action into full agonists and partial agonists (partially activated weaker in action than full agonists), but in the case of using a high dosage, full agonists act too powerfully and are liable to have a detrimental effect on the treatment of Parkinson's disease by levodopa. It has also been reported that, in actuality, the typical serotonin 1A receptor full agonist of 8-OH-DPAT exhibits an inhibitory action against the dyskinesia induced by levodopa in Parkinson's disease model marmosets and, with high dosages, a drop in the therapeutic effect by levodopa is recognized (Iravani M.M. et al., "In 1-methyl-4-phenyl-1,2,3,6- tetrahydropyridine-treated primates, the selective 5-hydroxytryptamine 1a agonist (R)-(+)-8-OHDPAT inhibits levodopa-induced dyskinesia but only with/increased motor disability", The Journal of Pharmacology and Experimental Therapeutics, 2006, 319, p. 1225-1234). Therefore, as shown in Example 3, rat hippocampuses were used to run a full agonist-partial agonist judgment test of SUN N4057 for serotonin 1A receptors. As a result, it became clear that SUN N4057 is a serotonin 1A receptor partial agonist. It was learned that, even if using a high dosage, there is little possibility of detrimentally influencing the treatment of Parkinson's disease by levodopa.

As is well known, Parkinson's disease is a progressive degenerative disorder. The symptoms deteriorate along with the advancing neurodegeneration. Delay in the advance of this neurodegeneration by the administration of a drug is one of the major unmet needs in Parkinson's disease. As opposed to this, in Parkinson's disease model monkeys, it is reported that a serotonin 1A receptor agonist delays the appearance of Parkinson's disease-like symptoms (Bezard E. et al., "5-HT1A receptor agonist-mediated protection from MPTP toxicity in mouse and macaque models of Parkinson's disease", Neurobiology of Disease, 2006, 23, p. 77-86). Further, it is known that a dopamine D3 receptor agonist has a nerve cell protective action (Joyce J.N. & Millan M.J., "Dopamine D3 receptor agonists for protection and repair in Parkinson's disease", Current Opinion in Pharmacology, 2007, 7, p. 100-105). Recently, the likelihood of having not only a protective action against nigrastriatal dopamine nerve cells specifically degenerating and being lost in Parkinson's disease, but a reparative action has been suggested (Van Kampen J.M. & Eckman C.B., "Dopamine D3 receptor agonist delivery to a model of Parkinson's disease restores the nigrostriatal pathway and improves locomotor behavior", The Journal of Neuroscience, 2006, 26, p. 7272-7280). SUN N4057 has a serotonin 1A receptor agonist (partial agonist) action and has an agonist action against dopamine D3 receptors as well. Therefore, these reports show that SUN N4057 is useful as a new drug for treatment of Parkinson's disease also having the effect of delaying the advance of symptoms of Parkinson's disease, based on the protective action on nigrastriatal dopamine nerve cells.

As shown in Example 4, the inventors treated rats with unilateral nigrostriatal dopamine nerve destruction generally used as Parkinson's disease animal models with repeated doses of levodopa, then used brain microdialysis to study the effects of SUN N4057 on the amount of dopamine release from rat striatum and rotational behavior of rats. As a result, SUN N4057 exhibited an inhibitory action against the increase in the amount of striatal dopamine release due to levodopa and delayed the peak time of the amount of dopamine release. The rotational behavior of rats decreased 30 minutes after levodopa dosage, but conversely was increased 150 to 210 minutes after levodopa administration. Further, judging from the amount of dopamine release in the striatum of normal rats studied using a similar method, the inhibitory action of SUN N4057 against dopamine release was believed to bring the amount of dopamine release due to levodopa closer to the normal concentration range. This shows that SUN N4057 has the action of inhibiting the increase of the amount of sudden striatal dopamine release due to levodopa in Parkinson's disease patients and keeping the dopamine in the normal concentration range longer.

As shown in Example 5, the inventors used rats with unilateral nigrostriatal dopamine nerve destruction generally used as Parkinson's disease animal models and treated them by repeated doses of levodopa so as to prepare advanced stage Parkinson's disease model rats and studied the effects on the hyperkinesia of the forelimbs appearing along with repeated doses of levodopa (abnormal behavior like dyskinesia in the clinical scene) and shortening of the rotational behavior duration (a phenomenon like wearing-off in the clinical scene). As a result, SUN N4057 exhibited the significant action for inhibiting hyperkinesia of the forelimbs and the action of extending the shortened rotational behavior duration. This shows that SUN N4057 is useful, as a drug for reducing the dyskinesia of motor complications associated with a treatment with levodopa of Parkinson's disease patients and reducing the off time and prolonging the duration of the pharmaceutical effect of levodopa (on time) not accompanied with dyskinesia.

As shown in Example 6, SUN N4057 exhibits an action alleviating response failure in Parkinson's disease model rats reported as resembling the on-off phenomenon in advanced stage Parkinson's disease patients. This suggests the SUN N4057 is useful as a drug for reducing the frequency of appearance of the on-off phenomenon of motor complications associated with a treatment with levodopa of Parkinson's disease patients.

Parkinson's disease is a progressive degenerative disorder of the nigrastriatal dopamine nerves. The condition is believed to deteriorate along with the advance of neurodegeneration. It is reported that motor complications due to levodopa long-term administration appear in about 40% of Parkinson's disease patients after the elapse of a treatment period of 4 to 6 years or more (see, the above-mentioned Ahlskog J.E. et al., Movement Disorders, 2001, 16, p. 448-458). As shown in Example 7, by repeated dosage of SUN N4057 together with the start of repeated doses of levodopa in Parkinson's disease model rat, the hyperkinesia of the forelimbs appearing along with repeated doses of levodopa (dyskinesia-like abnormal action in clinical studies) was inhibited. This shows that SUN N4057 also has the effect of delaying the onset of dyskinesia accompanying levodopa therapy in Parkinson's disease patients.

Further, the present inventors found that, as shown in Example 8, SUN N4057 significantly shortens the immobility period, an indicator of antidepressive action, in forced swimming tests without influencing the amount of spontaneous movement in rats and exhibits an increase in the swimming behavior period. A drug which significantly shortens the immobility period and increases the swimming behavior period in rat forced swimming tests is considered strong in effective of improving the mood (depressed mood and anxiety) in clinical tests (Katz M. M. et al., "Drug-induced actions on brain neurotransmitter systems and changes in behaviors and emotions of depressed patients", Neuropsychopharmacology, 1994, 11, p. 89-100). Furthermore, regarding the symptoms of depression in Parkinson's disease, it is reported that suicidal ideation, suicidal attempts, and other symptoms are rare, and therefore, this can be considered as a light depressed state or mood change different from the psychological disorder of major depression (Veazey C. et al., "Prevalence and treatment of depression in Parkinson's disease", The Journal of Neuropsychiatry and Clinical Neurosciences, 2005, 17, p. 310-323). Therefore, it is suggested from these that SUN N4057 promises a more suitable action against the depressed mood and anxiety observed in Parkinson's disease patients and has the additional effect of alleviating the depressed state and anxiety reported as the biggest factors lowering the quality of life of Parkinson's disease patients. Further, as a psychiatric symptom deteriorating along with advance of Parkinson's disease, hallucinations have been pointed out. Hallucinations are reportedly observed in about 20% of Parkinson's disease patients (Aarsland D. et al., "Range of neuropsychiatric disturbances in patients with Parkinson's disease", Journal of Neurology, Neurosurgery, and Psychiatry, 1999, 67, p. 492-496). The pathogenesis of hallucinations in Parkinson's disease patients is not clear, but it is interpreted that this is because the dopamine receptors of the cortex are continually stimulated unphysiologically by excessive dopamine, but the possibility of involvement of abnormalities in serotonin function has also been reported (Melamed E. et al., "Involvement of serotonin in clinical features of Parkinson's disease and complications of L-DOPA therapy", Advances in Neurology, 1996, 69, p. 545-550, Zoldan J. et al., "Psychosis in advanced Parkinson's disease: Treatment with ondansetron, a 5-HT3 receptor antagonist", Neurology, 1995, 45, p. 1305-1308). That is, it is hypothesized that hallucinations in Parkinson's disease patients are due to the excessive stimulus of the brain serotonin nervous system accompanying long-term levodopa use. This hypothesis is also supported by the clinical report that hallucinations of advanced stage Parkinson's disease patients are significantly inhibited by drugs inhibiting brain serotonin release (Melamed E. et al., "Involvement of serotonin in clinical features of Parkinson's disease and complications of L-DOPA therapy", Advances in Neurology, 1996, 69, p. 545-550). It is known that serotonin 1A receptor agonists inhibit brain serotonin release. These reports suggest the possibility of SUN N4057 inhibiting hallucinations accompanying levodopa therapy in advanced stage Parkinson's disease.

Next, as shown in Example 9, for the SUN N4057 analogs compound Ib and compound Ic as well, advanced stage Parkinson's disease model rats were prepared and the effects on hyperkinesia of the forelimbs (abnormal action like dyskinesia in clinical studies) and shortening of the rotational behavior duration appearing along with repeated doses of levodopa (phenomenon similar to wearing off in clinical studies) were studied. As a result, each compound exhibited significant action in inhibiting hyperkinesia of the forelimbs and action extending the shortened rotational behavior duration. This shows that the present invention compound is useful as a drug for alleviating the dyskinesia of motor complications associated with a treatment with levodopa of Parkinson's disease patients and wearing off phenomenon.

Further, as shown in Example 10, clinical tests studying the efficacy, safety and tolerability of SUN N4057 in Patients with motor complications associated with Parkinson's Disease suggests 1) the effect of increasing the percentage of "on" time (duration of the pharmacological effect of levodopa) without dyskinesia, 2) the effect of reducing the percentage of "off" time (the period where the effects of levodopa are insufficient and symptoms of Parkinson's disease (tremor, rigidity, akinesia, disorder of postural reflex) appear, and 3) the effect of improving the severity of the dyskinesia, without increasing "off" time or aggravation of Parkinson's disease symptoms.

The present compound may be jointly administered with a levodopa preparation. That is, it is sufficient to administer the present compound during levodopa therapy. For example, it is possible to administer it simultaneously with each levodopa dosage in a day or administer it once for several levodopa dosages. The levodopa preparation may be levodopa alone or a levodopa/peripheral dopa-decarboxylase inhibitor combination. As a peripheral dopa-decarboxylase inhibitor, carbidopa or benserazide may be mentioned.

Furthermore, according to the present invention, the present compound may, in addition to combined use with a levodopa preparation, use in combination one or both of a monoamine oxidase type B inhibitor and catechol-o-methyl transferase inhibitor for the treatment of Parkinson's disease. While not limited thereto, as the monoamine oxidase type B inhibitor, selegiline may be mentioned, while for the catechol-o-methyl transferase inhibitor, entacapone may be mentioned.

A composition containing the present compound, levodopa alone or a levodopa/peripheral dopa-decarboxylase inhibitor combination, and, furthermore, if necessary, one or both of a monoamine oxidase type B inhibitor and catechol-o-methyl transferase inhibitor is produced by a method well known in the art.

The present compound, when applied as a pharmaceutical, may be administered orally or non-orally. The preparation including the compound of the present invention may, for example, be any one of a tablet (including sugar-coated tablet and film-coated tablet), dispersion, fine subtilae, granules, capsule, liquid, suspension, injection, suppository, extended release preparation, etc. Note that, as suitable preparations for oral administration, depending on the Parkinson's disease patient, a tablet, dispersion, fine subtilae, granules, or capsule is desirable. These preparations are prepared according to ordinary methods (e.g., methods described in the Japan Pharmacopeia etc.)

Specifically, for the production method of a tablet, the pharmaceutical as it is may be uniformly mixed with an excipient, binder, disintegrating agent, or other suitable additives, formed into granules by a suitable method, then mixed with a lubricant and press molded or the pharmaceutical as it is may be uniformly mixed with an excipient, binder, disintegrating agent, or other suitable additives and directly press molded or may be uniformly mixed as is with granules prepared in advance or with the addition of suitable additives, then press molded. Further, this preparation may include, if necessary, a coloring agent, flavoring agent, etc. Furthermore, this preparation may be coated with a suitable coating agent.

For the production method of a capsule, according to the ordinary method, usually the present invention compound is mixed with an oral preparation vehicle and filled into a hard gelatin capsule, soft capsule, etc.

For the production method of an injection, a given amount of the pharmaceutical may be dissolved, suspended or emulsified in distilled water for injection use, physiological saline, Ringer's solution, etc. in the case of an aqueous solvent and normally vegetable oil etc. in the case of a non-aqueous solvent to obtain a given amount or a given amount of the pharmaceutical may be taken and sealed in a container for injection use.

As oral preparation vehicles, for example starch, mannitol, crystalline cellulose, sodium carboxymethyl cellulose, and other substances commonly used in the field of preparations may be used. As the injection vehicles, for example, distilled water, physiological saline, glucose solution, transfusions, etc. may be used. In addition, it is also possible to suitably add additives generally used for preparations.

The dosage of the present compound differs depending on the administration route, preparation form, number of dosages, age, body weight and degree of symptoms of the patient, etc., but in the case of oral administration, it is possible to administer, for a normal adult, 0.5 to 30 mg per day, preferably 0.5 to 15 mg, more preferably 1 to 5 mg, once a day or divided into two or more times. In the case of non-oral administration, it is possible to administer 1/10 of an amount or an equal amount to the case of oral administration, preferably 1/10 to 1/2 of the amount.

### EXAMPLES

The present invention will now be explained in further detail, based on Examples, but the scope of the present invention is by no means limited to these Examples. Further, for comparative study, sarizotan (1-[(2R)-3,4-dihydro-2H-chromen-2-yl]-N-{[5-(4-fluorophenyl)pyridin-3-yl]methyl}methanamine dihydrochloride), which is a serotonin 1A receptor agonist and which reportedly exhibits efficacy against motor complications accompanying levodopa therapy for Parkinson's disease in clinical test results, and the Example Compound 4 described in Japanese Patent Publication No. 2005-298402A (7-(1-{[5-(4-fluorophenyl) pyridin-3-yl]methyl}piperidin-3-yl)-1,3-benzoxazol-2(3H)-one dihydrochloride), which has an agonist action against both serotonin 1A receptors and dopamine D2 receptors and reportedly improves the dopamine stimulus score, without increasing the dyskinesia score much at all, in the evaluation of L-DOPA-induced dyskinesia behavior in Parkinson's disease model rats, were used.

### Example 1: Binding affinity tests on human serotonin 1A receptors, human dopamine D2L receptors, human dopamine D2S receptors and human dopamine D3 receptors

### 1-1. Binding affinity test on human serotonin 1A receptors

For the test, membrane specimens prepared from Chinese hamster ovary (CHO-K1) cells expressing human serotonin 1A receptors were used. To a 50 mM Tris-HCl buffer (pH 7.4) including 5 mM CaCl₂, 0.1% ascorbic acid, and 10 µg/mL saponin, [³H] 8-hydroxy-2-(di-n-propylamino)tetraline ([³H]8-OH-DPAT) (final concentration 1 nM), the test sample solution and the membrane specimen were added. The reaction solution was allowed to react at 25°C for 60 minutes, then the reaction solution was filtered with a cell harvester, the filtered filter paper was transferred to a measurement vial, a liquid scintillator was added and the receptor bond radioactivity remaining on the filter paper was determined by a liquid scintillation counter. The nonspecific bonds were defined as the amount of bonds in the presence of 10 µM serotonin.

The following formula was used to calculate the binding inhibition rate:
Binding inhibition rate (%)=100-100×{[amount of [³H] 8-OH-DPAT bonds in the presence of test sample]-[amount of [³H] 8-OH-DPAT bonds in the presence of 10 µM serotonin]}/{[amount of [³H] 8-OH-DPAT bonds in the absence of test sample]-[amount of [³H] 8-OH-DPAT bonds in the presence of 10 µM serotonin]}

From the binding inhibition rate, a regression formula was used to calculate the 50% inhibition concentration. Furthermore, the inhibition constant (Ki) was calculated.

### 1-2. Binding affinity test on human dopamine D2L receptors

For the test, membrane specimens prepared from Chinese hamster ovary (CHO) cells expressing human dopamine D2L receptors were used. To a 50 mM Tris-HCl (pH 7.4) buffer containing 1.4 mM ascorbic acid, 0.001% BSA, and 150 mM NaCl, [³H] spiperone (final concentration 0.16 nM), the test sample solution and a membrane specimen were added. The reaction solution was allowed to react at 25°C for 2 hours, then the reaction solution was filtered with a cell harvester, the filtered filter paper was transferred to a measurement vial, a liquid scintillator was added and the receptor bond radioactivity remaining on the filter paper was determined by a liquid scintillation counter. The nonspecific bonds were defined as the amount of bonds in the presence of 10 µM haloperidol.

The following formula was used to calculate the binding inhibition rate:
Binding inhibition rate (%)=100-100×{[amount of [³H] spiperone bonds in the presence of test sample]-[amount of [³H] spiperone bonds in the presence of 10 µM haloperidol]}/{[amount of [³H] spiperone bonds in the absence of test sample]-[amount of [³H] spiperone bonds in the presence of 10 µM haloperidol]}

From the binding inhibition rate, a regression formula was used to calculate the 50% inhibition concentration. Furthermore, the inhibition constant (Ki) was calculated.

### 1-3. Binding affinity test on human dopamine D2S receptors

For the test, membrane specimens prepared from Chinese hamster ovary (CHO) cells expressing human dopamine D2S receptors were used. To a 50 mM Tris-HCl (pH 7.4) buffer containing 1.4 mM ascorbic acid, 0.001% BSA, and 150 mM NaCl, [³H] spiperone (final concentration 0.16 nM), the test sample solution and a membrane specimen were added. The reaction solution was allowed to react at 25°C for 2 hours, then the reaction solution was filtered with a cell harvester, the filtered filter paper was transferred to a measurement vial, a liquid scintillator was added, and the receptor bond radioactivity remaining on the filter paper was determined with a liquid scintillation counter. The nonspecific bonds were defined as the amount of bonds in the presence of 10 µM haloperidol.

The following formula was used to calculate the binding inhibition rate:
Binding inhibition rate (%)=100-100×{[amount of [³H] spiperone bonds in the presence of test sample]-[amount of [³H] spiperone bonds in the presence of 10 µM haloperidol]}/{[amount of [³H] spiperone bonds in the absence of test sample]-[amount of [³H] spiperone bonds in the presence of 10 µM haloperidol]}

From the binding inhibition rate, a regression formula was used to calculate the 50% inhibition concentration. Furthermore, the inhibition constant (Ki) was calculated.

### 1-4. Binding affinity test on human dopamine D3 receptors

For the test, membrane specimens prepared from Chinese hamster ovary (CHO) cells expressing human dopamine D3 receptors were used. To a 50 mM Tris-HCl buffer (pH 7.4) containing 1.4 mM ascorbic acid, 0.001% BSA, and 150 mM NaCl, [³H] spiperone (final concentration 0.7 nM), the test sample solution and a membrane specimens were added. The reaction solution was allowed to react at 37°C for 2 hours, then the reaction solution was filtered with a cell harvester, the filtered filter paper was transferred to a measurement vial, a liquid scintillator was added and the receptor bond radioactivity remaining on the filter paper was determined by a liquid scintillation counter. The nonspecific bonds were defined as the amount of bonds in the presence of 25 µM S(-)-Sulpiride.

The following formula was used to calculate the binding inhibition rate:
Binding inhibition rate (%)=100-100×{[amount of [³H] spiperone bonds in the presence of test sample]-[amount of [³H] spiperone bonds in the presence of 25 µM S(-)-sulpiride]}/{[amount of [³H] spiperone bonds in the absence of tested substance]-[amount of [³H] spiperone bonds in the presence of sulpiride 25 µM S(-)-sulpiride]}

From the binding inhibition rate, a regression formula was used to calculate the 50% inhibition concentration. Furthermore, the inhibition constant (Ki) was calculated.

**Table I**

| | Ki (nM) | | |
|---|---|---|---|
| Receptor | SUN N4057 | Sarizotan | Example Compound 4 of Japanese Patent Publication No. 2005-298402A |
| Serotonin 1A | 0.0249 | 0.0293 | 2.67 |
| Dopamine D2L | 161 | 3.29 | 5.42 |
| Dopamine D2S | 162 | 2.56 | 6.34 |
| Dopamine D3 | 42.8 | 3.48 | 9.07 |

Table I shows the binding affinity with respect to human serotonin 1A receptors, human dopamine D2L receptors, human dopamine D2S receptors and human dopamine D3 receptor. For human serotonin 1A receptors, each of the test sample exhibited a strong binding affinity. SUN N4057 and sarizotan exhibited particularly strong binding affinity. On the other hand, for human dopamine (D2L, D2S and D3) receptors, sarizotan and Example Compound 4 of Japanese Patent Publication No. 2005-298402A exhibited comparatively strong binding affinity.

### Example 2: Study of agonist/antagonist activity for human serotonin 1A receptors, human dopamine D2L receptors, human dopamine D2S receptors, and human dopamine D3 receptors

For the tests, membrane specimens prepared from Chinese hamster ovary (CHO-K1) cells expressing human serotonin 1A receptors, Chinese hamster ovary (CHO) cells expressing human dopamine D2L receptors, Chinese hamster ovary (CHO) cells expressing human dopamine D2S receptors and Chinese hamster ovary (CHO) cells expressing human dopamine D3 receptors were used. In the agonist assays, for the serotonin 1A receptors, [³⁵S] GTPγS (final concentration 0.1 nM), a GDP solution (final concentration 3 µM), the test sample solution and a membrane specimen were added to a 20mM Hepes-NaOH buffer (pH 7.4) containing 100 mM NaCl, 3 mM MgCl₂ and 10 µg/mL saponin and allowed to react at 25°C for 30 minutes. Further, for the dopamine (D2L, D2S and D3) receptors, [³⁵S] GTPγS (i.e., final concentration 0.1 nM), a GDP solution (final concentration 3 µM), the test sample solution and a membrane specimen were added to a 20mM Hepes-NaOH buffer (pH 7.4) containing 100 mM NaCl, 10 mM MgCl₂, 1 mM DTT and 1 mM EDTA and allowed to react at 30°C for 15 minutes (in the case of D2L receptors) or for 30 minutes (in the cases of D2S and D3 receptors). Further, in the antagonist assays, except for adding to the reaction solutions 10 µM 5-carboxamide tryptamine (5-CT) (in the case of serotonin 1A receptors), 3 µM dopamine (in the cases of D2L and D2S receptors), or 0.1 µM dopamine (in the case of D3 receptors), operations were performed similar to the agonistic tests. After the end of the reaction, the reaction solution was filtered with a cell harvester, the filtered filter paper was transferred to a measurement vial, a liquid scintillator was added, and the radioactivity of the [³⁵S] GTPγS remaining on the filter paper was determined by a liquid scintillation counter. The human serotonin 1A receptor and human dopamine (D2L, D2S, and D3) receptor agonist/antagonist activities of the test sample were expressed as the rates of increase, when making the increase in [³⁵S] GTPγS bonds due to 10 µM 5-CT (in the case of serotonin 1A receptors), 3 µM dopamine (in the cases of D2L and D2S receptors), or 0.1 µM dopamine (in the case of D3 receptors) 100%. From the bond increasing rate, a regression formula was used to calculate the 50% reaction concentration (EC50) and 50% inhibition concentration (IC50).

**Table II-1**

| Serotonin 1A | Agonist assay | | Antagonist assay | |
|---|---|---|---|---|
| | EC50 (nM) | Emax | IC50 (nM) | Emax (%) |
| SUN N4057 | 2.41 | 100 | >100 | 3 |
| Sarizotan | 2.40 | 100 | >100 | 4 |
| Example Compound 4 of JP-2005-298402A | 108 | 100 | >20,000 | 14 |

**Table II-2**

| Dopamine D2L | Agonist assay | | Antagonist assay | |
|---|---|---|---|---|
| | EC50 (nM) | Emax (%) | IC50 (nM) | Emax (%) |
| SUN N4057 | >500 | 27 | >500 | 47 |
| Sarizotan | >500 | 2 | 13.3 | 101 |
| Example Compound 4 of JP-2005-298402A | >500 | 18 | 72.9 | 75 |

**Table II-3**

| Dopamine D2S | Agonist assay | | Antagonist assay | |
|---|---|---|---|---|
| | EC50 (nM) | Emax (%) | IC50 (nM) | Emax (%) |
| SUN N4057 | >500 | 41 | >500 | 25 |
| Sarizotan | >500 | 5 | 66.7 | 74 |
| Example Compound 4 of JP-2005-298402A | >500 | 27 | 17.9 | 69 |

**Table II-4**

| Dopamine D3 | Agonist assay | | Antagonist assay | |
|---|---|---|---|---|
| | EC50 (nM) | Emax (%) | IC50 (nM) | Emax (%) |
| SUN N4057 | 2.12 | 77 | >500 | 13 |
| Sarizotan | >500 | 14 | 181 | 65 |
| Example Compound 4 of JP-2005-298402A | 1.4 | 69 | >500 | 22 |

Tables II-1 to II-4 show the results of agonistantagonist assays for human serotonin 1A receptors, human dopamine D2L. receptors, human dopamine D2S receptors and human dopamine D3 receptors. For human serotonin 1A receptors, all of the test samples exhibited an agonist activity. SUN N4057 and sarizotan were particularly strong in activity. On the other hand, no antagonist activity was exhibited at all. For human dopamine D2L receptors and D2S receptors, none exhibited much agonist activity at all, while sarizotan and Example Compound 4 of Japanese Patent Publication No. 2005-298402A exhibited an antagonist activity. Further, for human dopamine D3 receptors, SUN N4057 and Example Compound 4 of Japanese Patent Publication No. 2005-298402A exhibited a strong agonist activity, but no antagonist activity was exhibited. On the other hand, sarizotan did not exhibit an agonist activity with respect to human dopamine D3 receptors, but exhibited a weak antagonist activity.

From these results, it became clear that SUN N4057 has a powerful agonist action against human serotonin 1A receptors, does not have an antagonist action against human dopamine D2 receptors, and has an agonist action against human dopamine D3 receptors.

### Example 3: Adenylate cyclase inhibition test via rat serotonin 1A receptors (full agonist-partial agonist judgment test)

In Example 3, 8-OH-DPAT and {2-[4-(4-pyrimidin-2-ylpiperidin-1-yl)butyl]-1,2-benzothiazole-3(2H)-one 1,1-deoxide} (hereinafter referred to as "ipsapirone") were used for comparison. 8-OH-DPAT is well known as a typical serotonin 1A receptor full agonist, while ipsapirone is well known as a typical serotonin 1A receptor partial agonist.

For the test, Wistar male rats supplied by Shimizu Laboratory Supplies were used (9 to 15 weeks old). Each rat was decapitated and the hippocampus was quickly removed. It was homogenized in 10 volumes of buffer (i.e., 25 mM Tris-HCl, 1 mM EGTA, 5 mM EDTA, 5 mM DTT, 300 mM sucrose, 100 KIU/ml aprotinin, pH 7.4). This was then centrifuged at 500xg, 4°C for 5 minutes, the supernatant was further centrifuged at 39,000xg, 4°C for 10 minutes, and the residue was used as the rat hippocampus membrane specimen. The rat hippocampus membrane specimen was diluted by a buffer to 20 to 90 µg/mL of protein, then 50 µL of this cell membrane suspension was added to a 200 µL assay buffer containing each test sample (25 mM Tris-HCl, 100 mM NaCl, 2 mM MgCl₂, 0.25 mM ATP,5 mM phosphocreatine, 10 µg/mL creatine phosphokinase, 0.2 mM IBMX, 10 µM GTP, 10 µM forskolin, pH 7.4) and allowed to react at 30°C for 5 minutes. 0.2N HCl was added in 250 µL to stop the reaction, then the cAMP produced was assayed by the radioimmunoassay method. From the concentration-reaction curve, a regression formula was used to calculate the 50% inhibition concentration (IC50) for the maximum reaction. Note that the adenylate cyclase activity was measured using as an indicator the amount of cAMP produced by the same enzyme. The rate of inhibition by the serotonin 1A receptor agonist was found using the amount of production due to forskolin (10 µM) stimulus as 100%.

SUN N4057 inhibited the adenylate cyclase activity stimulated by forskolin depending upon the concentration. The maximum inhibitory reaction was 20% (IC50=2.67 nM). The partial agonist ipsapirone and the full agonist 8-OH-DPAT also inhibited the adenylate cyclase activity depending upon the concentration. The maximum inhibitory reactions were 19% (IC50=38.95 nM) and 32% (IC50=14.82 nM) (see FIG. 1). Furthermore, these actions were completely antagonized by the serotonin 1A receptor selective antagonist WAY-100635 (100 nM).

From these results, it became clear that SUN N4057 acts as a partial agonist against serotonin 1A receptors of the serotonin nerve post-synapse membrane. Note that in Example 3, SUN N4057 is a partial agonist against serotonin 1A receptors, while in Example 2, full agonist-like results are obtained. This difference is believed to be due to the amount of expression of serotonin 1A receptors. In Example 2, for the cells, use was made of a line where the human serotonin 1A receptors were strongly expressed. Compared with membrane specimens prepared from rats, about 100 times the serotonin 1A receptors were present. There are reports that a serotonin 1A receptor partial agonist acts as a full agonist when the ratio of receptors with respect to the G proteins increases (Newman-Tancredi A et al., "Agonist and inverse agonist efficacy at human recombinant serotonin 5-HT1A receptors as a function of receptor: G-protein stoichiometry", Neuropharmacology, 1997, 36(4-5), p. 451-459), so it is interpreted that SUN N4057 exhibits full agonist-like results in the human serotonin 1A receptor expressing cells of Example 2.

### Example 4: Effect of single intraperitoneal administration of SUN N4057 on levodopa-induced striatal dopamine release in Parkinson's disease model rat (week 6 of repeated doses of levodopa) (test using brain microdialysis without anesthesia or physical restraint)

The Parkinson's disease treatment guidelines ("Japanese Society of Neurology Treatment Guidelines: Parkinson's Disease Treatment Guideline 2002", Ad Hoc Committee, Rinsho Shinkeigaku (Clinical Neurology), 2002, 42, p. 430-94) classify Parkinson's disease into the relatively mild early stage Parkinson's disease and the advanced stage Parkinson's disease where levodopa has been used for a long period and problems have arisen in the treatment. Therefore, the inventors treated rats with unilateral nigrostriatal dopamine nerve destruction generally used as Parkinson's disease animal models by repeated doses of levodopa, then used brain microdialysis to study the effects of single intraperitoneal administration of SUN N4057 on the amount of release of dopamine from the rat striatum and rotational behavior of rats.

For preparing Parkinson's disease model rats by unilateral nigrostriatal dopamine nerve destruction, Crl: CD (SD) male rats supplied by Charles River Japan (8 weeks old at start of test) were used. About 30 minutes before injection of 6-hydroxydopamine, the norepinephrine reuptake inhibitor desipramine (25 mg/kg) was intraperitoneally administered, then the rat head was fixed to a brain stereotaxic apparatus under anesthesia by intraperitoneal administration of pentobarbital (40 mg/kg). 6-hydroxydopamine (total amount 8 µg/4 µL) including ascorbic acid was dissolved in physiological saline, then was injected at two locations of the right medial forebrain bundle (at 1.8 mm posterior to the bregma, 2.0 mm right-lateral to the median line and 8.3 mm ventral to the surface of the skill and 4.5 mm to the rear from the bregma, 1.4 mm to the right from the midline, and a depth of 8.5 mm from the cranium) through a microinjection syringe at a flow rate of 1.0 µL/min over 4 minutes, then was allowed to stand for 5 minutes.

To confirm the success of dopamine nerve destruction after about 3 weeks from the above surgery, the rotational behavior of the rats after subcutaneous administration of the dopamine receptor agonist apomorphine (0.05 mg/kg) was measured using a rotational behavior-measuring equipment (Rotameter 6ch System, Muromachi Kikai Co., Ltd.). Rats rotating over 100 times in the opposite direction to the injection in the one hour right after apomorphine administration were judged to have unilateral nigrastriatal dopamine nerve destruction and were used for the subsequent tests.

Furthermore, from about one week later, the rats were given repeated intraperitoneal administration of levodopa (25 mg/kg) + peripheral dopa-decarboxylase inhibitor benserazide (10 mg/kg) (hereinafter referred to as "repeated doses of levodopa") (twice a day, from Monday to Friday) for 6 weeks.

For the brain microdialysis guide cannule insertion surgery, under anesthesia by pentobarbital (40 mg/kg intraperitoneal administration), the head of each rat was fixed on a brain stereotaxic apparatus, then a brain microdialysis guide cannule (Carnegie Medicin PC12 guide cannule) was inserted into the right striatum (0.5 mm in front of the bregma, 3.0 mm from the midline to the right, and 4.0 mm deep from the cranium) and dental cement was used to fix it to the rat skull. The next day, without anesthesia or physical restraint, a brain microdialysis probe (Carnegie Medicin PC12, outside diameter 0.5 mm, length of dialysis membrane 3.0 mm, cut off 20000 Daltons) was inserted through the guide cannule and Ringer's solution (Na⁺, 147 mM; K⁺, 4 mM; Ca²⁺, 2.3 mM; Cl⁻, 155.6 mM, pH 6.5) was conduced at flow rate of 1.5 µL/min (Carnegie Medicin CMA100). Starting from about 3 hours after insertion of the probe, the dialysate was collected every 20 minutes in a sample tube containing 5 µL of an antioxidant solution (0.1 M perchloric acid, 0.1 mM disodium EDTA, and 0.1 mM sodium metabisulfate). Five samples were obtained before levodopa dosage (100 minutes), and 15 samples were obtained after levodopa dosage (300 minutes). The obtained dialysate was immediately measured for dopamine concentration using a high performance liquid chromatography system with an electrochemical detector (LC-ECD). Further, every 30 minutes after levodopa administration, the number of rotations in 5 minutes to the side opposite to the 6-hydroxydopamine injection side was visually measured. SUN N4057 (3, 10 mg/kg) was intraperitoneally administered 20 minutes before administration of levodopa (25 mg/kg).

Note that the rate of biological utilization of SUN N4057 in the rats, with subcutaneous administration, was a good one of about 100%, but with intraperitoneal administration, was a low one of about 27%. Further, it became clear that this quickly was consumed peaking at 15 minutes after administration. For this reason, in intraperitoneal administration, a higher dosage was set than with subcutaneous administration.

As a result, due to the pre-intraperitoneal administration of SUN N4057 (3 and 10 mg/kg), an inhibitory action against the increase of the amount of striatal dopamine release due to levodopa was observed and the peak time of the amount of dopamine release was delayed (FIG. 2-A). Further, the rotational behavior of the rats decreased 30 minutes after levodopa administration, but conversely was observed to increase at 150 to 210 minutes after levodopa dosage (FIG. 2-B). It is known that the amount of dopamine release at the striatum of normal rats studied using a similar method was 9.24 ± 0.65(pg/20 min). Due to pre-administration of SUN N4057, the amount of dopamine release induced by levodopa is believed to have approached the normal concentration range. Therefore, it is suggested that SUN N4057 has the action of inhibiting the increase of the amount of sudden striatal dopamine release induced by levodopa in Parkinson's disease model rats and keeping the dopamine in the normal range of concentration for a longer time.

### Example 5: Test evaluating behavior in hyperkinesia of the forelimbs and shortening of rotational behavior duration induced by repeated doses of levodopa using Parkinson's disease model rats

In rats with unilateral nigrostriatal dopamine nerve destruction generally used as Parkinson's disease animal models, it became clear that by administering levodopa by repeated dosage for about 5 weeks, behavior resembling the symptoms of advanced stage Parkinson's disease appeared. That is, in Parkinson's disease model rats prepared in the same way as in Example 4, along with repeated doses of levodopa, abnormal behavior such as involuntary extending of the forelimb or opening or closing of the hand, up-and-down movement of the wrist, choreiform tremor in the forelimb on the opposite side of microinjection of 6-hydroxydopamine was observed (FIG. 3). This abnormal behavior is reported by Steece-Collier et al. (Steece-Collier K. et al., "Embryonic mesencephalic grafts increase levodopa-induced forelimb hyperkinesia in parkinsonian rats", Movement Disorders, 2003, 18, p. 1442-1454) as hyperkinesia of the forelimbs. This appears dependent on the period of repeated doses of levodopa and reportedly is similar to clinical dyskinesia. Further, if comparing the rotational behavior on day 1 of levodopa dose and week 5 of repeated doses, the rotational behavior duration (period exhibiting 20% or more of maximum number of rotations when measuring number of rotations every 5 minutes for each rat) is shortened (FIG. 4). Bibbiani et al. (Bibbiani F. et al., "Serotonin 5-HT1A agonist improves motor complications in rodent and primate parkinsonian models", Neurology, 2001, 57, p. 1829-1834) report that in Parkinson's disease model rats, along with repeated doses of levodopa, a shortening of the rotational behavior duration is observed and that this phenomenon resembles the wearing off phenomenon in clinical studies.

Therefore, in the same way as Example 4, the inventors prepared Parkinson's disease model rats, treated the model rats with repeated doses of levodopa (twice a day, Monday to Friday) for 5 weeks, then observed them for hyperkinesia of the forelimbs at the times of 30 minutes and 1 and 2 hours after levodopa dosage for 2 minutes each time and recorded the periods where they exhibited abnormal behavior (seconds). The rotational behaviors of the same rats were measured using a rotational behavior measuring system for every 5 minutes for 4 hours from right after levodopa intraperitoneal administration, finding the period during which the individual rats exhibited 20% or more of the maximum number of rotations in accordance with the report of Bibbiani (Bibbiani F. et al., Neurology, 2001, 57, p. 1829-1834), and defining this as the rotational behavior duration.

Further, based on the results of the hyperkinesia of the forelimbs and rotational behavior duration, the inventors assigned the rats to the groups so that no significant difference occurred among them, then compared and studied the effects of the tested substances on these behaviors resembling symptoms of advanced stage Parkinson's disease patients. That is, they treated each group by repeated doses of levodopa and repeated dosage of the tested substance for about 2 weeks (Monday to Friday) and observed the hyperkinesia of the forelimbs and rotational behavior after levodopa administration. Note that the SUN N4057 was subcutaneously administered right before levodopa administration, the sarizotan was orally administered about 20 minutes before levodopa dosage in accordance with above-mentioned known reports (Bibbiani F. et al., Neurology, 2001, 57, p. 1829-1834), and the Example Compound 4 of Japanese Patent Publication No. 2005-298402A was subcutaneously administered about 30 minutes before levodopa dosage in accordance with the above-mentioned Japanese Patent Publication No. 2005-298402A. Further, for the solvent, for SUN N4057 and Example Compound 4 of Japanese Patent Publication No. 2005-298402A, physiological saline was used. Sarizotan is poor in solubility, so physiological saline containing 0.3% Tween80 was used.

As a result, with SUN N4057 (0.03 mg/kg), hyperkinesia of the forelimbs 1 hour after levodopa dosage was significantly inhibited, while with SUN N4057 (0.1 mg/kg), hyperkinesia of the forelimbs 1 and 2 hours after levodopa dosage was significantly inhibited (FIG. 5). Regarding the rotational behavior after levodopa administration, with SUN N4057 (0.03, 0.1 mg/kg), the end of the rotational behavior was significantly prolonged (FIG. 6-A). The rotational behavior duration found from the number of rotations was also significantly prolonged (FIG. 6-B). Therefore, it was suggested that SUN N4057 is effective against the dyskinesia and wearing off phenomenon in advanced stage Parkinson's disease patients.

Due to sarizotan 5 mg/kg, a tendency of the hyperkinesia of the forelimbs to be inhibited 1 hour after levodopa dosage (p=0.099) was observed (FIG. 7). Further, by sarizotan 1 and 5 mg/kg, a delay in the start of rotational behavior due to levodopa dosage was observed (FIG. 8-A). Regarding the rotational behavior duration found from the number of rotations, with sarizotan 5 mg/kg, significant shortening was observed (FIG. 8-B). Therefore, it was suggested that in advanced stage Parkinson's disease patients, sarizotan had a low likelihood of inhibiting motor complications without reducing the therapeutic effect of levodopa.

With the Example Compound 4 of Japanese Patent Publication No. 2005-298402A (1, 3 mg/kg), no action was exhibited again hyperkinesia of the forelimbs of the rats after levodopa dosage (FIG. 9). However, regarding the rotational behavior after levodopa administration, due to the Example Compound 4 of Japanese Patent Publication No. 2005-298402A (i.e., 1, 3 mg/kg), the rotational behavior started earlier and the end of the rotational behavior was prolonged (FIG. 10-A). Regarding the rotational behavior duration found from the number of rotations as well, with the 3 mg/kg administration group, significant prolongation was observed (FIG. 10-B). Therefore, with Example Compound 4 of Japanese Patent Publication No. 2005-298402A, there is a possibility of an effect on the wearing off phenomenon in the motor complications induced by levodopa, but it was suggested there was no effect on dyskinesia.

### Example 6: Test on rate of incidence of rotational behavior induced by levodopa dosage using Parkinson's disease model rats

Papa et al. (Papa, S.M. et al., "Motor fluctuations in levodopa treated parkinsonian rats: relation to lesion extent and treatment duration", Brain Research, 1994, 662, p. 69-74) report that in Parkinson's disease model rats, along with repeated doses of levodopa, rats not exhibiting rotational behavior even if administered levodopa are irregularly observed, calls this phenomenon "response failure", and reports that this phenomenon resembles the on-off phenomenon in advanced stage Parkinson's disease patients.

Therefore, the inventors prepared Parkinson's disease model rats in the same way as in Example 4 and treated the rats by repeated doses of levodopa (twice a day, Monday to Friday) for 7 weeks. The presence or absence of the rotational behavior of each rat was recorded at approximately 1 hour after the administration of levodopa during the repeated dosing period. As a result, at week 1 after start of repeated doses of levodopa, almost all rats exhibited rotational behavior after levodopa administration, but from week 2 of repeated doses of levodopa, rats were observed not exhibiting rotational behavior even if administering levodopa. Therefore, from weeks 1 to 5 of repeated doses of levodopa, the inventors recorded the presence of any rotational behavior of the rats each time, then studied the effects of the tested substances from week 6 of repeated doses of levodopa by implanting an Alzet® osmotic pump filled with SUN N4057 or physiological saline (2ML4, volume 2 ml, flow rate 2.5 µl/hr) subcutaneously at the rats. In the 2 weeks after that, the inventors similarly continued to record the presence of any rotational behavior of the rats 1 hour after levodopa administration.

As a result, in the physiological saline sustained subcutaneous administration group, the rate of incidence of rotational behavior tended to fall along with repeated doses of levodopa, but in the SUN N4057 sustained subcutaneous administration group, an action on recovery of the rate of incidence of rotational behavior was observed (FIG. 11). Note that the measured values of the concentration of SUN N4057 in the plasma of each group measured after the end of the behavioral experiments were 5.3 ± 0.7 ng/mL in the SUN N4057 low dosage sustained subcutaneous administration group and 14.3 ± 2.9 ng/mL in the high dosage sustained subcutaneous administration group.

From these results, it was suggested that SUN N4057 promises an alleviating effect on the on-off phenomenon of advanced stage Parkinson's disease patients.

### Example 7: Test of effect inhibiting induction of hyperkinesia of the forelimbs using Parkinson's disease model rats

By repeated doses of levodopa to Parkinson's disease model rats, hyperkinesia of the forelimbs (abnormal action similar to dyskinesia in clinical studies) was observed (see FIG. 3). Therefore, it was studied whether it is possible to inhibit the induction of hyperkinesia of the forelimbs by repeated dosage of the tested substance from the start along with the start of repeated doses of levodopa.

Parkinson's disease model rats were prepared in the same way as in Example 4, administered physiological saline or SUN N4057 (3 mg/kg) by intraperitoneal administration along with the start of repeated doses of levodopa for 3 weeks (Monday to Friday), and the rats were observed for hyperkinesia of the forelimbs by a method similar to Example 5. The tested substance was intraperitoneally administered about 20 minutes before levodopa administration. After this, a 3 week withdrawal period (during which tested substance was not administered and only repeated doses of levodopa was performed) was set and the same rats were observed for hyperkinesia of the forelimbs by the same method.

As a result, by intraperitoneal administration of SUN N4057 (3 mg/kg) together with the start of repeated doses of levodopa, the appearance of hyperkinesia of the forelimbs in Parkinson's disease model rats was reduced. Compared with the results after the withdrawal period, a significant inhibitory effect was observed (FIG. 12-A,B). Therefore, the likelihood of SUN N4057 exhibiting an effect of inhibiting or delaying the onset of dyskinesia accompanying long-term administration of levodopa in Parkinson's disease patients was suggested.

### Example 8: Test evaluating anti-depressive action using forced swimming method

For the test, Crl: CD(SD) male rats supplied by Charles River Japan were used (6 weeks old at start of test). For the forced swimming test, the method of the improved type reported by Lucki et al. (Lucki I., "The forced swimming test as a model for core and component behavioral effects of antidepressant drugs", Behavioural Pharmacology, 1997, 8, p. 523-532; Cryan J.F. et al., "Assessing antidepressant activity in rodents: recent developments and future needs", TRENDS in Pharmacological Sciences, 2002, 23, p. 238-245) was used. On day 1, each rat was placed in a cylinder filled with water (height 45 cm x diameter 30 cm, water depth 30 cm, water temperature 23°C, water depth set to depth by which back legs and tail of rat will not touch the bottom). The rat was taken out after 15 minutes. At this time, the immobility period (seconds) of minutes 10-15 (5 minutes) was recorded. Based on the results, the rats were assigned to five groups so that there would be no difference among the groups. On day 2, each rat was placed in a cylinder filled with water the same as day 1 and the immobility period (seconds), swimming behavior period (seconds), and climbing behavior period (seconds) of minutes 0-5 (5 minutes) were measured. Note that the immobility period was defined as the minimum extent of movement for the rat to hold its head above the water, the swimming behavior as the swimming behavior circling around the inside of the cylinder in the horizontal direction, and the climbing behavior as the climbing behavior moving the forelimbs in the vertical direction along the cylinder walls to try to climb them. The tested substance was intraperitoneally administered 15 minutes after the end of swimming on day 1, and 4 hours and 30 minutes before swimming on day 2. Further, for the amount of locomotor activity, a measuring system (shape 48.8 x 48.8 x 30 cm, vertical 8 fractions, horizontal 8 fractions, for total of 64 fractions, Biomedica) set in a blacked out soundproof box was used to record the amount of locomotor activity of the rats at minutes 0-15 (15 minutes). Note that the dosages and timings of administration of the tested substances were set in the same way as the forced swimming test and the substance were intraperitoneally administered 24 hours, 4 hours, and 30 minutes before measurement of the amount of locomotor activity.

As a result, SUN N4057 (0.3, 1 and 3 mg/kg) exhibited significant shortening of the immobility period in accordance with the dosage. The desipramine (10 mg/kg), well known as an antidepressant, also exhibited significant shortening of the immobility period (FIG. 13-A). In addition to the immobility period, the climbing and swimming behavior periods were measured. As a result, due to desipramine (10 mg/kg), a significant increase in the climbing behavior period was observed (FIG. 13-B), while with SUN N4057 (1, 3 mg/kg), a significant increase in the swimming behavior period was observed (FIG. 13-C). The amount of locomotor activity was observed to decline due to desipramine (10 mg/kg) at minutes 0-5 (5 minutes). However, with SUN N4057 (0.3, 1, and 3 mg/kg), there was no significant change (FIG. 13-D).

The forced swimming test is being widely used as a method of pre-clinical evaluation since antidepressants have the action of shortening the immobility period. Further, it is reported that, in a modified forced swimming test, the immobility period is reduced by any drug exhibiting activation of the norepinephrine nervous system or serotonin nervous system, but the climbing behavior period increases by drugs exhibiting activation of the norepinephrine nervous system and the swimming behavior period increases by drugs exhibiting activation of the serotonin nervous system (Cryan JF et al., "Noradrenergic lesions differentially alter the antidepressant-like effects of reboxetine in a modified forced swim test", European Journal of Pharmacology, 2002, 436, p. 197-205). In clinical studies, antidepressants activating the norepinephrine nervous system are considered to have a strong effect in increasing motivation and stimulating stagnant nervous movement (slow movement, anguished expression, behavioral inactivity, and fewer words), while antidepressants activating the serotonin nervous system are considered to have strong effects in improving mood (depressed mood and anxiety) (see the above-mentioned, Katz M.M. et al., Neuropsychopharmacology, 1994, 11, p. 89-100).

SUN N4057 significantly shortens the immobility period and increases the swimming behavior period without influencing the amount of locomotor activity. Therefore, it is suggested that SUN N4057 has an antidepressive action and furthermore is likely to exhibit an effect improving mood (depressed mood and anxiety) - pointed out as being the biggest factor impairing the quality of life of Parkinson's disease patients.

### Example 9: Test evaluating behavior for compound Ib and compound Ic in hyperkinesia of the forelimbs and shortening of rotational behavior duration induced by repeated doses of levodopa using Parkinson's disease model rats

Using the same method as Example 5, compound Ib and compound Ic were observed for hyperkinesia of the forelimbs and rotational behavior after levodopa administration. In the same way as Example 4, Parkinson's disease model rats were prepared and the rats treated by repeated doses of levodopa (twice a day, Monday to Friday) for 5 weeks and measured for hyperkinesia of the forelimbs and rotational behavior. Further, based on the results of the hyperkinesia of the forelimbs and rotational behavior duration, the rats were assigned to the groups so as to prevent any significant difference between them, then the effects of the tested substances on these behaviors resembling symptoms of advanced stage Parkinson's disease patients were compared and studied. That is, each group was treated by repeated doses of levodopa and repeated dosage of the tested substance for about 2 weeks (Monday to Friday) and was observed for hyperkinesia of the forelimbs and rotational behavior after levodopa administration. Note that compound Ib (0.3, 1 mg/kg) and compound Ic (0.3, 1 mg/kg) were both intraperitoneally administered right before levodopa administration. For the solvent, physiological saline was used.

As a result, due to compound Ib (0.3, 1 mg/kg), hyperkinesia of the forelimbs 30 minutes and 1 hour after levodopa dosage was significantly inhibited (FIG. 14-A). The rotational behavior duration found from the number of rotations also tended to be prolonged (FIG. 14-B). Further, with compound Ic (0.3, 1 mg/kg), with 0.3 mg/kg, the hyperkinesia of the forelimbs 30 minutes after levodopa dosage was significantly inhibited, while with 1.0 mg/kg, hyperkinesia of the forelimbs 30 minutes and 1 hour after levodopa dosage was significantly inhibited (FIG. 15-A). The rotational behavior duration found from the number of rotations was also significantly prolonged with 1.0 mg/kg (FIG. 15-B). Therefore, the likelihood of SUN N4057 analog compound Ib exhibiting an effect of alleviation of dyskinesia in advanced stage Parkinson's disease patients and the compound Ic exhibiting such an effect on the dyskinesia and wearing off phenomenon is suggested.

### Example 10: Clinical test studying efficacy, safety and tolerability of SUN N4057 in patients with motor complications associated with Parkinson's Disease

### Method:

The efficacy of SUN N4057 on motor complications accompanying levodopa therapy in Parkinson's disease patients was studied in randomized, double-blind, placebo-controlled, multicenter, international, preliminary study. The patients aged 40 to 85 years, having idiopathic Parkinson's disease fulfilling the United Kingdom Parkinson's Disease Society (UK PDS) brain bank diagnostic criteria, having a presumed or confirmed diagnosis of Parkinson's disease in Stages 2 to 4 on the Hoehn & Yahr classification scale when in an "off" state, having a diagnosis of Parkinson's disease for at least 5 years, and being on a stable regimen of levodopa (L-dopa)/carbidopa for 30 days prior to the administration of the tested drug were enrolled into the study.

After providing their informed consent, the subjects were screened in 9 to 22 days before scheduled date of start of administration. During the screening phase, the patients continued to take their usual regimen of antiparkinsonian drugs including combinations of levodopa and carbidopa or combined use of single drugs. Patients which, due to the drug therapy, exhibited a therapeutic effect improving their scores (points) by at least 25% in the Unified Parkinson's Disease Rating Scale (UPDRS), Part 3 (testing of motor functions) compared with before administration were judged as qualified patients.

At the baseline (8 ± 2 days before scheduled date of start of administration), the subjects returned to the clinic for a baseline assessment of motor fluctuations over an 8-hour period. They received the first morning dose of the patient's usual anti-Parkinson's disease drugs (including combination of levodopa and carbidopa or combined use of single drugs), and the 8 hour evaluation began approximately 1 hour after taking the initial treatment. Each hour for 8 hours, the patient were assessed and it was recorded whether the patient was in the "on" (without dyskinesia), "on with dyskinesia," or "off" motor states. The Unified Parkinson's Disease Rating Scale (UPDRS) Part III and the Abnormal Involuntary Movement Scale AIMS were also completed each hour over the 8-hour observation period.

The subjects judged eligible after the screening and baseline evaluation were randomly assigned in a 3:1 ratio (3 active:1 placebo) to receive SUN N4057 or matching placebo. The subjects received continuous intravenous infusion of SUN N4057 (target plasma concentration of 30 ng/mL) or a placebo for 12 hours a day for 2 consecutive days. The dose regimen was selected to reach the target plasma concentration 1 hour after the start of administration and to be maintained for 11 hours. The mean dosage of SUN N4057 for the patients was 13.123 mg on day 1 and 9.550 mg on day 2.

On day 1, motor assessments (recording of "on" or "off" periods), the Unified Parkinson's Disease Rating Scale (UPDRS) Part III, and the Abnormal Involuntary Movement Scale (AIMS) were conducted immediately prior to dosing with regular antiparkinsonian medications. After the first morning dose of the regular antiparkinsonian medication was administered, intravenous infusion of SUN N4057 or placebo was started. For 8 hours from the start of infusion, motor assessments, including the UPDRS Part III, AIMS, and recording of "on" and "off" periods were to be completed hourly.

### Results:

No remarkable difference in demographics and baseline characteristics were observed between the treatment groups.

The percentage of on time without dyskinesia in the SUN N4057 group, the percentage of on time with dyskinesia, and the percentage of off time were, evaluated on day 2, 41.0%, 49.3%, and 9.7%. The percentage of on time without dyskinesia increased by 22.2 points in the comparison with baseline (41.0-18.8), while the percentage of off time decreased 11.1 points in the comparison with baseline (9.7-20.8) (Table III).

The percentage of on time without dyskinesia, the percentage of on time with dyskinesia, and the percentage of off time in the placebo group were, evaluated on day 2, 12.5%, 64.3%, and 23.2%. The percentage of on time without dyskinesia did not show any change in the comparison with the baseline (12.5-12.5), while the percentage of off time increased by 14.3 points in the comparison with the baseline (23.2-8.9) (Table III).

**Table III. Motor States of Placebo Group and SUN N4057 Group Before and After Administration**

| | Before administration (baseline) | | | After administration (day 2) | | |
|---|---|---|---|---|---|---|
| | Percentage of on time without dyskinesia (%) | Percentage of on time with dyskinesia (%) | Percentage of off time (%) | Percentage of on time without dyskinesia (%) | Percentage of on time with dyskinesia (%) | Percentage of off time (%) |
| Placebo group (n=7) | 12.5 | 78.6 | 8.9 | 12.5 | 64.3 | 23.2 |
| SUN N4057 group (n=18) | 18.8 | 60.4 | 20.8 | 41.0 | 49.3 | 9.7 |

Further, the percentage of responder, defined as patients where the dyskinesia evaluated by the Abnormal Involuntary Movement Scale was improved in severity or suppressed to zero without prolongation of off time, was significantly higher in the SUN N4057 group (i.e., 56%: day 2) compared with the placebo group (i.e., 0%: day 2) (p=0.02).

### In the evaluation by the Unified Parkinson's Disease Rating Scale (UPDRS) Part 3, on day 2 of administration, the placebo group exhibited a 0.9 point decline in comparison with the baseline, while the SUN N4057 group exhibited a 4.4 point decline in comparison with the baseline.

In the clinical study, no serious adverse events or deaths were reported.

In conclusion, results of clinical study on SUN N4057 suggest the following:
A) The most preferable time for Parkinson's disease patients, that is, the period when they get good levodapa response on the motor functioning assessment(on time) without dyskinesia, increased in the SUN N4057 group after administration of the drug compared with the baseline. Furthermore, this increase was larger compared with the placebo group.
B) The period where the effect of levodopa is insufficient and the symptoms of Parkinson's disease (tremors, rigidity, akinesia and disorder of postural reflex) appear, that is, the "off" time, was reduced in the SUN N4057 group after administration of the drug compared with the baseline. In the placebo group, it was increased after administration of the placebo compared with the baseline.
C) In responder analysis using as indicators the Abnormal Involuntary Movement Scale (AIMS) and reduction of the off time, SUN N4057 exhibited significant treatment effects.
D) In the evaluation by the Unified Parkinson's Disease Rating Scale (UPDRS) Part 3, the SUN N4057 group exhibited somewhat higher improvement compared with the placebo group.

Therefore, it was considered that SUN N4057 might be effective in the treatment of motor complications associated with L-dopa therapy for the treatment of Parkinson's disease.

## Claims

1. A drug for alleviating motor complications associated with a treatment with levodopa for Parkinson's disease comprising a compound having the formula (I): 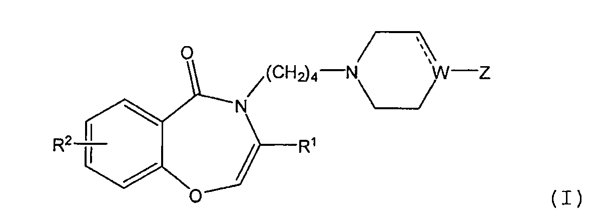 wherein R¹ indicates a hydrogen atom, chlorine atom or C₁ to C₄ alkyl group, R² indicates a hydrogen atom, halogen atom, methyl group or methoxy group, W indicates a nitrogen atom, CH or carbon atom, the dotted line indicates the absence of a bond when W is a nitrogen atom or CH and indicates the presence of a bond when W is a carbon atom, Z indicates a phenyl group, pyridyl group, or pyrimidinyl group which is unsubstituted or substituted with 1 to 3 substituent groups selected from the group consisting of a methyl group, methoxy group and halogen atom
or a pharmacologically acceptable salt thereof or their hydrates.

2. A drug as claimed in claim 1, wherein, in the formula (I), R¹ indicates a chlorine atom, R² indicates a hydrogen atom, W indicates a carbon atom, the dotted line indicates the presence of a bond and Z indicates a pyridyl group or pyrimidinyl group which is unsubstituted or substituted with a methyl group.

3. A drug as claimed in claim 1, wherein the compound having the formula (I) is 3-chloro-4,5-dihydro-4-{4-[4-(2-pyridyl)1-1,2,3,6-tetrahydropyridin-1-yl]butyl}-1,4-benzoxazepin-5-one.

4. A drug as claimed in any one of claims 1 to 3, which reduces dyskinesia in motor complications associated with a treatment with levodopa for Parkinson's disease.

5. A drug as claimed in any one of claims 1 to 3, which reduces the off time in the treatment with levodopa of Parkinson's disease.

6. A drug as claimed in any one of claims 1 to 3, which prolongs the duration of pharmacological effect of levodopa (i.e., on time) not accompanied with dyskinesia in the treatment with levodopa of Parkinson's disease.

7. A drug as claimed in any one of claims 1 to 3, which reduces the frequency of appearance of the on-off phenomenon in motor complications associated with levodopa therapy for Parkinson's disease.

8. A drug as claimed in any one of claims 1 to 3, which suppresses the increase in the amount of levodopa required for the treatment accompanying with the advance of the symptoms of Parkinson's disease.

9. A drug as claimed in any one of claims 1 to 3, which reduces the number of dosages of levodopa per day required for the treatment in levodopa therapy of Parkinson's disease.

10. A drug for inhibiting or delaying the advance of symptoms of Parkinson's disease comprising a compound having the formula (I): 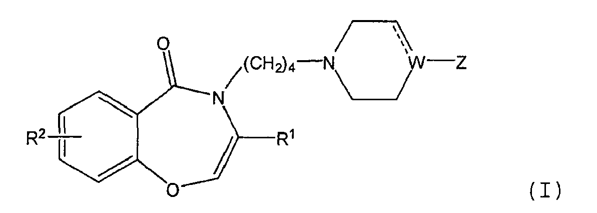 wherein R¹ indicates a hydrogen atom, chlorine atom or C₁ to C₄ alkyl group, R² indicates a hydrogen atom, halogen atom, methyl group or methoxy group, W indicates a nitrogen atom, CH or carbon atom, the dotted line indicates the absence of a bond when W is a nitrogen atom or CH and indicates the presence of a bond when W is a carbon atom, Z indicates a phenyl group, pyridyl group, or pyrimidinyl group, which is unsubstituted or substituted with 1 to 3 substituent groups selected from the group consisting of a methyl group, methoxy group and halogen atom
or a pharmacologically acceptable salt thereof or their hydrates.

11. A drug as claimed in claim 10, wherein, in the formula (I), R¹ indicates a chlorine atom, R² indicates a hydrogen atom, W indicates a carbon atom, the dotted line indicates the presence of a bond and Z indicates a pyridyl group or pyrimidinyl group, which is unsubstituted or substituted with a methyl group.

12. A drug as claimed in claim 10, wherein the compound having the formula (I) is 3-chloro-4,5-dihydro-4-{4-[4-(2-pyridyl)-1,2,3,6-tetrahydropyridin-1-yl]butyl}-1,4-benzoxazepin-5-one.

13. A drug for delaying the onset of motor complications associated with a treatment with levodopa for Parkinson's disease comprising a compound having the formula (I): 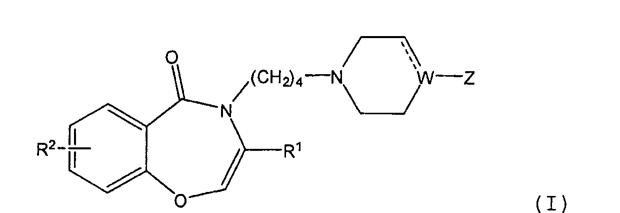 wherein R¹ indicates a hydrogen atom, chlorine atom or C₁ to C₄ alkyl group, R² indicates a hydrogen atom, halogen atom, methyl group or methoxy group, W indicates a nitrogen atom, CH or carbon atom, the dotted line indicates the absence of a bond when W is a nitrogen atom or CH and indicates the presence of a bond when W is a carbon atom, Z indicates a phenyl group, pyridyl group or pyrimidinyl group, which is unsubstituted or substituted with 1 to 3 substituent groups selected from the group consisting of a methyl group, methoxy group and halogen atom
or a pharmacologically acceptable salt thereof or their hydrates.

14. A drug as claimed in claim 13, wherein, in the formula (I), R¹ indicates a chlorine atom, R² indicates a hydrogen atom, W indicates a carbon atom, the dotted line indicates the presence of a bond and Z indicates a pyridyl group or pyrimidinyl group which is unsubstituted or substituted with a methyl group.

15. A drug as claimed in claim 13, wherein the compound having the formula (I) is 3-chloro-4,5-dihydro-4-{4-[4-(2-pyridyl)-1,2,3,6-tetrahydropyridin-1-yl]butyl}-1,4-benzoxazepin-5-one.

16. A drug for alleviating a psychiatric symptom accompanying with advanced stage Parkinson's disease comprising a compound having the formula (I): 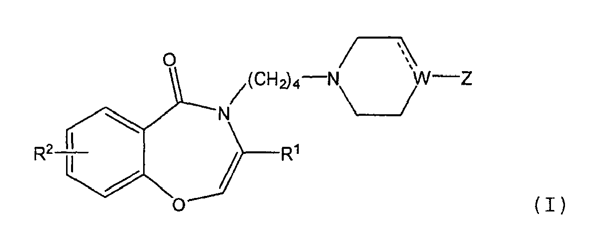 wherein R¹ indicates a hydrogen atom, chlorine atom or C₁ to C₄ alkyl group, R² indicates a hydrogen atom, halogen atom, methyl group or methoxy group, W indicates a nitrogen atom, CH or carbon atom, the dotted line indicates the absence of a bond when W is a nitrogen atom or CH and indicates the presence of a bond when W is a carbon atom, Z indicates a phenyl group, pyridyl group or pyrimidinyl group which is unsubstituted or substituted with 1 to 3 substituent groups selected from the group consisting of a methyl group, methoxy group and halogen atom
or a pharmacologically acceptable salt thereof or their hydrates.

17. A drug as claimed in claim 16, wherein, in the formula (I), R¹ indicates a chlorine atom, R² indicates a hydrogen atom, W indicates a carbon atom, the dotted line indicates the presence of a bond and Z indicates a pyridyl group or pyrimidinyl group which is unsubstituted or substituted with a methyl group.

18. A drug as claimed in claim 16, wherein the compound having the formula (I) is 3-chloro-4,5-dihydro-4-{4-[4-(2-pyridyl)-1,2,3,6-tetrahydropyridin-1-yl]butyi}-1,4-benzoxazepin-5-one.

19. A drug as claimed in any one of claims 16 to 18, wherein the psychiatric symptom accompanied with advanced stage Parkinson's disease is depression, anxiety or hallucinations.

20. A drug as claimed in any one of claims 1 to 19, which is used in combination with at least one drug selected from peripheral dopa-decarboxylase inhibitors, monoamine oxidase type B inhibitors and catechol-O-methyl transferase inhibitors.

21. A pharmaceutical composition for treatment of Parkinson's disease comprising
(a) a compound having the formula (I): 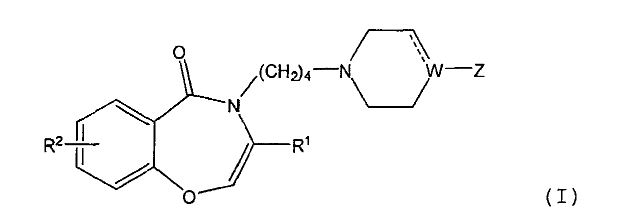 wherein R¹ indicates a hydrogen atom, chlorine atom or C₁ to C₄ alkyl group, R² indicates a hydrogen atom, halogen atom, methyl group or methoxy group, W indicates a nitrogen atom, CH or carbon atom, the dotted line indicates the absence of a bond when W is a nitrogen atom or CH and indicates the presence of a bond when W is a carbon atom, Z indicates a phenyl group, pyridyl group, or pyrimidinyl group which is unsubstituted or substituted with 1 to 3 substituent groups selected from the group consisting of a methyl group, methoxy group and halogen atom
or a pharmacologically acceptable salt thereof or their hydrates and
(b) levodopa.

22. A pharmaceutical composition for treatment of Parkinson's disease comprising
(a) a compound having the formula (I): 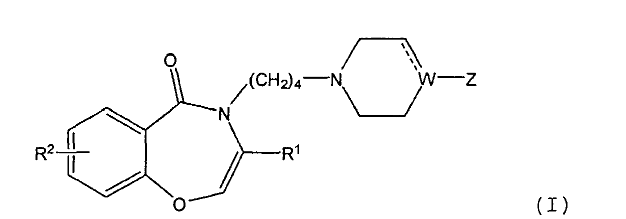 wherein R¹ indicates a hydrogen atom, chlorine atom or C₁ to C₄ alkyl group, R² indicates a hydrogen atom, halogen atom, methyl group or methoxy group, W indicates a nitrogen atom, CH or carbon atom, the dotted line indicates the absence of a bond when W is a nitrogen atom or CH and indicates the presence of a bond when W is a carbon atom, Z indicates a phenyl group, pyridyl group or pyrimidinyl group which is unsubstituted or substituted with 1 to 3 substituent groups selected from the group consisting of a methyl group, methoxy group and halogen atom
or a pharmacologically acceptable salt thereof or their hydrates,
(b) levodopa and
(c) one or more drugs selected from peripheral dopa-decarboxylase inhibitors, monoamine oxidase type B inhibitors, and catechol-o-methyl transferase inhibitors.

23. A composition as claimed in claim 21 or 22 wherein, in the formula (I), R¹ indicates a chlorine atom, R² indicates a hydrogen atom, W indicates a carbon atom, the dotted line indicates the presence of a bond and Z indicates a pyridyl group or pyrimidinyl group which is unsubstituted or substituted with a methyl group.

24. A composition as claimed in claim 21 or 22
wherein the compound having the formula (I) is 3-chloro-4,5-dihydro-4-{4-[4-(2-pyridyl)-1,2,3,6-tetrahydropyridin-1-yl]butyl}-1,4-benzoxazepin-5-one.
